# EUROPÄISCHE PATENTSCHRIFT

(11) **EP 2 756 066 B1**
(45) Veröffentlichungstag und Bekanntmachung des Hinweises auf die Patenterteilung: **14.11.2018**
(21) Anmeldenummer: 12745863.6
(22) Anmeldetag: 10.08.2012
(51) Int. Cl.: C11D 3/50, C11D 17/00, C11D 3/37

(54) **MIKROKAPSELHALTIGES MITTEL**
AGENT CONTAINING MICROCAPSULES
PRODUIT CONTENANT DES MICROCAPSULES

(30) Priorität: 12.09.2011 DE 102011082496
(43) Veröffentlichungstag der Anmeldung: 23.07.2014
(73) Patentinhaber: Henkel AG & Co. KGaA, 40589 Düsseldorf (DE)
(72) Erfinder: BAUER, Andreas, 41564 Kaarst (DE); HUCHEL, Ursula, 50670 Köln (DE); URLICHS, Stefan, 45139 Essen (DE); MATERNE, Manuela, 41564 Kaarst (DE); LAST, Klaus, 37520 Osterode (DE); MUES, Daniel, 31633 Leese (DE)
(74) Vertreter: Viering, Jentschura & Partner mbB Patent- und Rechtsanwälte
(86) Internationale Anmeldenummer: PCT/EP2012/065640
(87) Internationale Veröffentlichungsnummer: WO 2013/037575

(56) Entgegenhaltungen:
- WO-A1-2011/075556
- WO-A2-2010/102830
- US-A1- 2002 064 541

## Beschreibung

Die Erfindung liegt auf dem Gebiet der kosmetischen Mittel, der Reinigungsmittel und Textilbehandlungsmittel, die Mikrokapseln enthalten, sowie Verfahren zur Freisetzung von Aktivstoffen aus diesen Mikrokapseln bei der Anwendung besagter Mittel.

Eine Vielzahl von kosmetischen Mitteln, Reinigungsmitteln und Textilbehandlungsmitteln enthalten empfindliche Inhaltsstoffe, wie z.B. Riechstoffe oder Pflanzenextrakte. Nachteilig ist, dass solche Inhaltsstoffe, die in solchen Mitteln eingesetzt werden, häufig schon bei der Lagerung und/oder vor dem gewünschten Anwendungszeitpunkt ihre Aktivität verlieren oder zumindest stark reduziert werden und zwar beispielsweise durch chemische Reaktionen infolge Interaktion mit anderen Bestandteilen der jeweiligen Mittel und/oder durch physikalische Einflüsse. Aus diesem Grund ist eine Verkapselung bestimmter Inhaltsstoffe vorteilhaft.

Es existieren bereits zahlreiche kommerzielle Verkapselungssysteme, die auf natürlichen oder künstlichen Polymeren basieren. Diese können einen Wirkstoff oder dessen Lösung umschließen und dann in der Hülle physikalisch oder chemisch vernetzt werden oder durch einen Koazervationsprozess mit einem anderen Polymer ausgefällt werden. Aus dem Stand der Technik sind Mikrokapseln bekannt, die als Kernmaterial flüssige, feste oder gasförmige Stoffe enthalten können. Als Material für die Kapselwände sind beispielsweise Phenol-Formaldehyd-Polymere, MelaminFormaldehyd-Polymere, Polyurethan, Gelatine, Polyamide oder Polyharnstoffe gebräuchlich.

Kosmetische Mittel, Reinigungsmittel und Textilbehandlungsmittel, welche Mikrokapseln enthalten, sind als solche bekannt. Insbesondere haben sich Mikrokapseln aus Melamin-FormaldehydHarzen in diesen Mitteln bewährt, da diese besonders stabil sind. Derartige formaldehyd-haltige Kapsel(mischungen) werden beispielsweise in der WO 2011/075556 A1 beschrieben. Problematisch ist jedoch, dass die bei der Herstellung dieser Mikrokapseln anfallenden Kapseldispersionen grundsätzlich noch restlichen freien Formaldehyd umfassen, dessen Anwesenheit bei der Weiterverarbeitung bzw. im Endprodukt, das an den Verbraucher abgegeben wird, unerwünscht ist. Es gibt in der Patenliteratur daher Vorschläge, den Formaldehydgehalt durch Einsatz von Formaldehydfängern abzusenken. So beschreiben EP 0 383 358 und US 4,918,317 unterschiedliche Mikrokapseln und Verfahren zu deren Herstellung mit dem Ziel, überschüssigen Formaldehyd mit Hilfe von Formaldehydfängern zu entfernen.

Durch den Zusatz der genannten Formaldehydfänger zur fertigen Mikrokapseldispersion bzw. bei der Herstellung der Mikrokapseldispersion wird regelmäßig der Formaldehydgehalt der Dispersion gesenkt. Oft lässt sich jedoch der Formaldehydgehalt von Produkten, die derartige Mikrokapseldispersionen enthalten oder damit behandelt wurden auch bei Zugabe großer Mengen an Formaldehydfänger nicht unterhalb eine bestimmte Grenze senken.

Es ist daher von allgemeinem Interesse, mikrokapselhaltige kosmetische Zusammensetzungen sowie Reinigungsmittel oder Textilbehandlungsmittel bereitzustellen, welche Mikrokapseln enthalten, die mit einem möglichst niedrigen Formaldehydeintrag einhergehen bzw. bei denen bevorzugt auf die Verwendung von Formaldehyd für Mikrokapseln ganz verzichtet wird. US 2002/064541 A1 beschreibt beispielsweise Kapseln deren Kapselwände ein anorganisches Polymer enthalten.

Andere Formaldehyd-freie Mikrokapseln werden beispielsweise aus einem Harz, aus aromatischem Alkohol und aldehydischer Komponente hergestellt. Die WO 2010/102830 A2 beschreibt Formaldehyd-freie Mikrokapseln, deren Kapselwände ein Harz umfassen, welches durch Umsetzung a) mindestens eines aromatischen Alkohols oder dessen Ether oder Derivat und b) mindestens einer aldehydischen Komponente, die mindestens zwei C-Atome pro Molekül aufweist, und c) optional mindestens eines (Meth)-Acrylat-Polymeren erhältlich ist. Ferner ist eine Mikrokapseldispersion, die ein oder mehrere Mikrokapseln enthält, offenbart. Einige Formaldehyd-freie Mikrokapseln aus einem Harz, aus aromatischem Alkohol und aldehydischer Komponente, verursachen fast keine Farbveränderung im Endprodukt und zeigen nach längerer Lagerung lediglich eine schwache Sedimentation der Kapseln, weisen teilweise allerdings, wenn sie mit einem Aktivstoff beladen wurden, bei längerer Lagerung nur noch einen schwachen Boosteffekt (Freigabe des Aktivstoffs nach Reibung) auf. Andere Formaldehyd-freie Mikrokapseln aus einem Harz, aus aromatischem Alkohol und aldehydischer Komponente, haben wiederum den Vorteil, dass sie auch bei längerer Lagerung noch einen sehr starken Boosteffekt erzielen, jedoch schwächere Ergebnisse bezüglich Sedimentation bei Lagerung und Farbveränderungen des Endproduktes aufweisen. Es besteht daher weiterhin Bedarf an Formaldehyd-freien Mikrokapseln, die gute Eigenschaften bezüglich Farbechtheit des Mittels, Lagerstabilität und Wirkstoffabgabe besitzen.

Aufgabe der vorliegenden Erfindung war es daher, Formaldehyd-freie Mikrokapseln mit guten Boosteigenschaften und guter Lagerstabilität zu entwickeln, welche nicht zu einer Farbbeeinträchtigung des Endprodukts führen.

Überraschenderweise hat sich gezeigt, dass die Kombination ausgewählter, unterschiedlicher Formaldehyd-freier Mikrokapseln zu synergistischen Effekten bezüglich Boosteffekt, Lagerstabilität und Farbgebung im Endprodukt führt.

Die Aufgabe wird daher gelöst durch Mittel zum Waschen, Reinigen, Konditionieren, Pflegen und/oder Färben von harten oder weichen Oberflächen, umfassend
i) mindestens eine oberflächenaktive Substanz, sowie
ii) ein Gemisch aus mindestens einer ersten und einer zweiten Mikrokapsel, deren Kapselwände jeweils ein Harz umfassen, welches durch Umsetzung
   a) mindestens eines aromatischen Alkohols oder dessen Ether oder Derivate mit
   b) mindestens einer aldehydischen Komponente, die mindestens zwei C-Atome pro Molekül aufweist, und
   c) in Gegenwart mindestens eines (Meth)Acrylat-Polymeren, erhältlich ist,
   wobei sich die erste und die zweite Mikrokapsel in mindestens einer der umgesetzten Komponenten a) und/oder b) voneinander unterscheiden und wobei der mindestens eine aromatische Alkohol ausgewählt ist aus Resorcin und/oder Phloroglucin.

Des Weiteren betrifft die Erfindung ein Verfahren zur Freisetzung eines Aktivstoffes aus einer Mikrokapsel, welche sich auf einer Oberfläche befindet, wobei die Mikrokapsel durch Kontakt eines Mittels zum Waschen, Reinigen, Konditionieren, Pflegen und/oder Färben von harten oder weichen Oberflächen, umfassend
i) mindestens eine oberflächenaktive Substanz, sowie
ii) ein Gemisch aus mindestens einer ersten und einer zweiten Mikrokapsel, die jeweils mindestens einen Aktivstoff enthalten und deren Kapselwände ein Harz umfassen, welches durch Umsetzung
   d) mindestens eines aromatischen Alkohols oder dessen Ether oder Derivate mit
   e) mindestens einer aldehydischen Komponente, die mindestens zwei C-Atome pro Molekül aufweist, und
   f) in Gegenwart mindestens eines (Meth)Acrylat-Polymeren, erhältlich ist,
   wobei sich die erste und die zweite Mikrokapsel in mindestens einer der umgesetzten Komponenten a) und/oder b) voneinander unterscheiden und wobei der mindestens eine aromatische Alkohol ausgewählt ist aus Resorcin und/oder Phloroglucin,
auf die Oberfläche aufgebracht wird und anschließend das Freisetzen des Aktivstoffes durch mechanische Krafteinwirkung erfolgt. Bevorzugt erfolgt das Freisetzen des Aktivstoffes durch Reibung.

Bevorzugt enthalten die Mittel zum Waschen, Reinigen, Konditionieren, Pflegen und/oder Färben von harten oder weichen Oberflächen, Mikrokapseln in Mengen von 0,0001 bis 50 Gew.-%, vorzugsweise 0,01 bis 20 Gew.-%, und insbesondere 0,1 bis 5 Gew.-%, bezogen auf das gesamte Mittel.

Bevorzugt werden die erste und die zweite Mikrokapsel im Verhältnis von 1 : 99 bis 99 : 1, besonders bevorzugt im Verhältnis von 1 : 50 bis 50 : 1 und insbesondere bevorzugt im Verhältnis von 9:1 bis 1:9 Gew.-% eingesetzt.

Bei den Mitteln zum Waschen, Reinigen, Konditionieren, Pflegen und/oder Färben von harten oder weichen Oberflächen im Sinne dieser Anmeldung handelt es sich um Wasch-, Reinigungs-, Nachbehandlungs- und/oder kosmetische Mittel.

Die erfindungsgemäßen Mittel werden zum Waschen, Reinigen, Konditionieren, Pflegen und/oder Färben von harten oder weichen Oberflächen verwendet. Harte Oberflächen im Sinne dieser Anmeldung sind dabei Fenster, Spiegel und weitere Glasoberflächen, Oberflächen aus Keramik, Kunststoff, Metall oder auch Holz sowie lackiertes Holz, die sich in Haushalt und Gewerbe finden, etwa Badkeramik, Koch- und Speisegeschirr, Küchenoberflächen oder Fußböden. Weiche Oberflächen im Sinne dieser Anmeldung sind textile Flächengebilde, Haut sowie Haare.

Mittel zum Waschen von harten oder weichen Oberflächen im Sinne dieser Anmeldung sind Textilwaschmittel, z.B. in Form von Pulvern, Granulaten, Perlen, Tabletten, Pasten, Gelen, Tüchern, Stücken oder Flüssigkeiten vorliegenden Formulierungen.

Mittel zum Reinigen von harten oder weichen Oberflächen im Sinne dieser Anmeldung umfassen alle Reiniger für harte oder weiche Oberflächen, insbesondere Geschirrspülmittel, Allzweckreiniger, WC-Reiniger, Sanitärreiniger sowie Glasreiniger, Zahncremes, Hautwaschmittel, wie Duschgele, oder Haarwaschmittel.

Mittel zum Konditionieren von harten oder weichen Oberflächen im Sinne dieser Anmeldung sind Weichspüler, Duftspüler, Konditioniertücher für die Anwendung im Wäschetrockner, Hygienespüler, Deodorantien, Antitranspirantien, Haarkonditioniermittel, Stylingmittel und/oder Haarfestigungsmittel.

Mittel zur Pflege von harten oder weichen Oberflächen im Sinne dieser Anmeldung sind Textilpflegemittel, Haarpflegemittel oder Hautbehandlungsmittel, wie beispielsweise Cremes, Lotionen oder Gele.

Mittel zum Färben von harten oder weichen Oberflächen im Sinne dieser Anmeldung sind Haarfärbe- und Haartönungsmittel und Mittel zum Aufhellen keratinischer Fasern.

Die Mittel zum Waschen, Reinigen, Konditionieren, Pflegen und/oder Färben von harten oder weichen Oberflächen haben den Vorteil, dass sie allenfalls einen ganz niedrigen Formaldehydgehalt aufweisen, da ihre Herstellung allenfalls mit einem ganz niedrigen, insbesondere aber mit gar keinem, Formaldehydeintrag einhergeht. Die Mittel zum Waschen, Reinigen, Konditionieren, Pflegen und/oder Färben von harten oder weichen Oberflächen ermöglichen die gezielte Freisetzung von Aktivstoffen, insbesondere von Riechstoffen und/oder Pflanzenextrakten, welche in den Kapseln gespeichert sind. Die Kapseln sind innerhalb der Mittel-Matrix stabil und können durch gezielten Reiz, insbesondere mechanische Krafteinwirkung, geöffnet werden. Unter mechanische Krafteinwirkung im Sinne der vorliegenden Erfindung wird jede Art der Krafteinwirkung auf die Mikrokapsel verstanden, wie z.B. Scherkräfte, Druck und/oder Reibung. Bei der Anwendung des Mittels, z.B. bei der Textilwäsche oder der Hautreinigung, lagern sich die Mikrokapseln auf der harten oder weichen Oberflächen ab und können nach der Trocknung der Oberfläche z.B. durch Reibung leicht geöffnet werden. Auf diese Weise gelingt eine gezielte Freisetzung des/der Aktivstoff(e), so dass das Leistungsprofil des gesamten Mittels erhöht wird. Dabei kommt insbesondere der Duftwirkung eine besondere Bedeutung zu, da die Produktleistung in vielen Fällen vom Verbraucher proportional zum Wohlgeruch beurteilt wird. Die Freisetzung der Aktivstoffe, insbesondere von Riechstoffen und/oder Pflanzenextrakten, kann aber auch auf diffusivem Weg erfolgen, bei dem die Aktivstoffe, insbesondere Duftstoffe und/oder Pflegestoffe, durch das polymere Hüllmaterial wandern und dann langsam freigesetzt werden. Das vorliegende Mittel zum Waschen, Reinigen, Konditionieren, Pflegen und/oder Färben von harten oder weichen Oberflächen ermöglicht eine langanhaltende Aktivstofffreisetzung, insbesondere eine langanhaltende Beduftung und Pflege von harten oder weichen Oberflächen sowie eine gezielte Aktivstofffreisetzung, insbesondere die Freisetzung von Riechstoffen und/oder Pflanzenextrakten, auch nach langen Zeitabständen durch Einsatz der mikroverkapselten Aktivstoffe.

In einer bevorzugten Ausführungsform handelt es sich bei der Oberfläche um eine textile Oberfläche. Wenn es sich bei der Oberfläche um eine textile Oberfläche handelt ist insbesondere bevorzugt, wenn das Mittel zum Waschen, Reinigen, Konditionieren, Pflegen und/oder Färben von harten oder weichen Oberflächen ein Wasch-, Reinigungs- oder Nachbehandlungsmittel ist.

In einer weiteren Ausführungsform handelt es sich bei der Oberfläche um eine Körperstelle, insbesondere um Haut und/oder Haare. Wenn es sich bei der Oberfläche um eine Körperstelle, insbesondere um Haut und/oder Haare, handelt ist bevorzugt, wenn das Mittel zum Waschen, Reinigen, Konditionieren, Pflegen und/oder Färben von harten oder weichen Oberflächen eine kosmetische Zusammensetzung ist.

Die Mikrokapseln enthalten insbesondere eine Flüssigkeit, vorzugsweise einen oder mehrere flüssige Aktivstoffe. Diese Aktivstoffe umfassen beispielsweise
i. Riechstoffe (Parfümöle)
ii. Pflegestoffe (z.B. Pflanzenextrakte)
iii. flüssige Wasch- und Reinigungsmittelinhaltsstoffe, wie vorzugsweise Tenside, insbesondere Niotenside, Silikonöle, Paraffine
iv. flüssige nicht-pharmazeutische Additive oder Wirkstoffe, z.B. Öle wie beispielsweise Mandelöl oder kühlende Substanzen, sowie
v. Mischungen daraus.

Es ist allerdings am meisten bevorzugt, dass Riechstoffe (Parfümöle) und/oder Pflegestoffe in den Mikrokapseln enthalten sind. Die Begriffe "Riechstoffe" und "Duftstoffe" werden im Rahmen dieser Erfindung synonym verwendet.

Unter Pflegestoffe im Sinne der vorliegenden Erfindung sind solche Stoffe zu verstehen, die einen pflegenden Effekt auf Textilien, die menschliche Haut und/oder Haare ausüben. Darunter fallen insbesondere Planzenextrakte. Besonders bevorzugt ist Aloe Vera Extrakt.

Der Kontakt der Pflegestoff-enthaltenden Mikrokapseln mit der Haut und/oder den Haaren kann entweder durch direkten Kontakt der Haut und/oder den Haaren mit einer kosmetischen Zusammensetzung umfassend Pflegestoff-enthaltende Mikrokapseln und/oder durch Übertragung der Pflegestoffe und/oder der Pflegestoff-enthaltenden Mikrokapseln durch Textilien, die solche Mikrokapseln auf der Oberfläche tragen, geschehen.

In einer bevorzugten Ausführungsform enthalten die erste und/oder die zweite Mikrokapsel Aktivstoff. Die erste und die zweite Mikrokapsel können dabei unabhängig voneinander jeweils denselben oder unterschiedlichen Aktivstoff enthalten. Folglich kann sich der Aktivstoff, enthalten in der ersten Mikrokapsel, von dem Aktivstoff, enthalten in der zweiten Mikrokapsel, ganz oder teilweise unterscheiden. Die erste und die zweite Mikrokapsel können auch denselben Aktivstoff enthalten. Es kann auch nur die erste Mikrokapsel Aktivstoff enthalten während die zweite Mikrokapsel keinen Aktivstoff enthält und umgekehrt.

In einer bevorzugten Ausführungsform handelt es sich bei dem eingesetzten Aktivstoff um Aloe Vera Extrakt. In einer besonders bevorzugten Ausführungsform enthält mindestens eine der eingesetzten Mikrokapseln zu mindestens 50 Gew.-% Pflanzenextrakt, insbesondere Aloe Vera Extrakt, bezogen auf das Gesamtgewicht des in der jeweiligen Mikrokapsel enthaltenen Aktivstoffes.

Im Folgenden werden die einsetzbaren Mikrokapseln genauer beschrieben.

Als aromatische Alkohole a) sind im Rahmen der vorliegenden Erfindung Resorcin und/oder Phloroglucin ausgewählt, aromatische Verbindungen, bei denen zwei oder drei freie Hydroxylgruppen unmittelbar aromatisch in meta Stellung gebunden sind.

In einer weiteren Ausführungsform der vorliegenden Erfindung enthalten die kosmetischen Mittel Mikrokapseln, bei deren Herstellung der aromatische Alkohol a) als Ether eingesetzt wird, wobei der Ether in einer bevorzugten Ausführungsform ein Derivat der jeweiligen freien Form des umzusetzenden aromatischen Alkohols a) ist. Der freie Alkohol kann dabei ebenso vorhanden sein; dann liegt demnach eine Mischung vor. Für diesen Fall kann das molare Verhältnis zwischen der freien Form des erfindungsgemäß umzusetzenden aromatischem Alkohols und der genannten zusätzlichen Komponente (Etherform eines aromatischen Alkohols) zwischen 0:100, bevorzugt 1:1, oder 1:2 oder 1:4 betragen.

Der Vorteil der Mischung des aromatischen Alkohols mit einer Ether-Form ist darin begründet, dass damit die Reaktivität des Systems beeinflussbar ist. Insbesondere lässt sich mit der geeigneten Auswahl des Verhältnisses ein System schaffen, dessen Reaktivität in einem ausgewogenen Verhältnis zu der Lagerstabilität des Systems steht. Als Derivate der aromatischen Alkohole sind Ester bevorzugt.

In einer bevorzugten Ausführungsform unterscheiden sich die erste und die zweite Mikrokapsel in der umgesetzten Komponenten a) voneinander.

Durch die Verwendung der aromatischen Alkohole a) Phloroglucin und/oder Resorcin werden besonders stabile Mikrokapseln erhalten. Mischungen aus Mikrokapseln, enthaltend je einen dieser beiden Alkohole, erzielten besonders gute Effekte bei Betrachtung des Boosteffekts, des Sedimentationsverhaltens und der Farbgebung im fertigen Endprodukt, selbst bei längerer Lagerung. In einer bevorzugten Ausführungsform enthält daher die erste Mikrokapsel Phloroglucin und die zweite Mikrokapsel Resorcin als aromatischen Alkohol a).

Besonders hervorragende Ergebnisse wurden erzielt, wenn die erste Mikrokapsel, enthaltend Phloroglucin, und die zweite Mikrokapsel, enthaltend Resorcin, in einem Verhältnis von 1:30 bis 1:3, bevorzugt von 1:19 bis 1:4 und insbesondere bevorzugt von 1:9 bis 1:4 eingesetzt wurden.

Als Aldehyde b) mit mindestens 2 C-Atomen sind gemäß der vorliegenden Erfindung sowohl aliphatische als auch aromatische Aldehyde bevorzugt. Besonders bevorzugte Aldehyde sind einer oder mehrere ausgewählt aus der folgenden Gruppe Valeraldehyd, Capronaldehyd, Caprylaldehyd, Decanal, Succindialdehyd, Cyclohexancarbaldehyd, Cyclopentancarbaldehyd, 2-Methyl-1-propanal, 2-Methylpropionaldehyd, Acetaldehyd, Acrolein, Aldosteron, Antimycin A, 8'-Apo-β-caroten-8'-al, Benzaldehyd, Butanal, Chloral, Citral, Citronellal, Crotonaldehyd, Dimethylaminobenzaldehyd, Folinsäure, Fosmidomycin, Furfural, Glutaraldehyd, Glycerinaldehyd, Glykolaldehyd, Glyoxal, Glyoxylsäure, Heptanal, 2-Hydroxybenzaldehyd, 3-Hydroxybutanal, Hydroxymethylfurfural, 4-Hydroxynonenal, Isobutanal, Isobutyraldehyd, Methacrolein, 2-Methylundecanal, Mucochlorsäure, N-Methylformamid, 2-Nitrobenzal-dehyd, Nonanal, Octanal, Oleocanthal, Orlistat, Pentanal, Phenylethanal, Phycocyanin, Piperonal, Propanal, Propenal, Protocatechualdehyd, Retinal, Salicylaldehyd, Secologanin, Streptomycin, Strophanthidin, Tylosin, Vanillin, Zimtaldehyd und Mischungen daraus. Besonders stabile Mikrokapseln wurden mit den bevorzugten aldehydischen Komponenten b) Glutardialdehyd und/oder Succindialdehyd erhalten.

Im Sinne der vorliegenden Erfindung kann die aldehydische Komponente mindestens ein oder zwei, besonders bevorzugt zwei, drei oder vier, insbesondere zwei freie Aldehydgruppen pro Molekül, aufweisen, wobei es bevorzugt ist, wenn als aldehydische Komponente mindestens Glyoxal, Glutar- und/oder Succindialdehyd vorliegt, besonders bevorzugt ist Glutardialdehyd.

In den erfindungsgemäß einsetzbaren Mikrokapseln kann das molare Verhältnis von a) dem mindestens einen aromatischen Alkohol oder (Ether oder Derivat davon), zu b) der mindestens einen aldehydischen Komponente im Allgemeinen zwischen 1:1 und 1:5, besonders bevorzugt zwischen 1 zu 2 und 1 zu 3 und ganz besonders bevorzugt bei Resorcin bei etwa 1 zu 2,6 liegen. Das Gewichts-Verhältnis der Komponenten a) + b) zu c), d.h. das Verhältnis der Gewichts-Summe von a) + b)) zum Gewicht der Komponente c) liegt im Allgemeinen zwischen 1:1 und 1:0,01 besonders bevorzugt zwischen 1:0,2 und 1:0,05.

In einer besonderen Ausführungsform unterscheiden sich die erste und die zweite Mikrokapsel in der umgesetzten Komponenten b) voneinander.

Bei den verwendeten (Meth)Acrylat-Polymeren kann es sich um Homo- oder Copolymere von Methacrylat-Monomeren und/oder Acrylat-Monomeren handeln. Der Begriff "(Meth)Acrylat" bezeichnet in dieser Erfindung sowohl Methacrylate wie Acrylate. Die (Meth)Acrylat-Polymere sind z.B. Homo-oder Copolymere, bevorzugt Copolymere, eines oder mehrerer polar funktionalisierter (Meth)Acrylat-Monomere, wie sulfonsäuregruppen-haltige, carbonsäuregruppen-haltige, phosphorsäuregruppen-haltige, nitrilgruppen-haltige, phoshonsäure-haltige, ammoniumgruppen-haltige, Amingruppen-haltige oder nitratgruppen-haltige (Meth)Acrylat-Monomere. Die polaren Gruppen können dabei auch in Salzform vorliegen. Die (Meth)Acrylat-Polymere eigenen sich als Schutzkolloide und können vorteilhaft bei der Herstellung von Mikrokapseln eingesetzt werden.

(Meth)Acrylat-Copolymere können beispielsweise aus zwei oder mehr (Meth)Acrylat-Monomeren bestehen (z.B. Acrylat + 2-Acrylamido-2-methyl-propansulfonsäure) oder aus einem oder mehreren (Meth)Acrylat-Monomeren und einem oder mehreren von (Meth)Acrylat-Monomeren verschiedenen Monomeren (z.B. Methacrylat + Styrol).

Beispiele für (Meth)Acrylat-Polymere sind Homopolymere von sulfonsäuregruppen-haltigen (Meth)Acrylaten (z.B. 2-Acrylamido-2-methyl-propansulfonsäure oder dessen Salze (AMPS), kommerziell erhältlich als Lupasol®PA 140, BASF), oder deren Copolymere, Copolymere von Acrylamid und (Meth)Acrylsäure, Copolymere von Alkyl-(Meth)Acrylaten und N-Vinylpyrrolidon (kommerziell erhältlich als Luviskol®K15, K30 oder K90, BASF), Copolymere von (Meth)Acrylaten mit Polycarboxylaten oder Polystyrolsulfonaten, Copolymere von (Meth)Acrylaten mit Vinylethern und/oder Maleinsäureanhydrid, Copolymere von (Meth)Acrylaten mit Ethylen und/oder Maleinsäureanhydrid, Copolymere von (Meth)Acrylaten mit Isobutylen und/oder Maleinsäureanhydrid, oder Copolymere von (Meth)Acrylaten mit Styrol-Maleinsäureanhydrid.

Bevorzugte (Meth)Acrylat-Polymere sind Homo- oder Copolymere, bevorzugt Copolymere, von 2-Acrylamido-2-methyl-propansulfonsäure oder dessen Salzen (AMPS). Bevorzugt sind Copolymere von 2-Acrylamido-2-methyl-propansulfonsäure oder dessen Salzen, z.B. Copolymere mit einem oder mehreren Comonomeren aus der Gruppe der (Meth)Acrylate, der Vinylverbindungen wie Vinylester oder Styrole, der ungesättigten Di- oder Polycarbonsäuren wie Maleinsäureester, oder der Salze von Amylverbindungen oder Allylverbindungen. Nachfolgend werden bevorzugte Comonomere für AMPS genannt, diese Comonomere können jedoch auch mit anderen polar funktionalisierten (Meth)Acrylat-monomeren copolymerisiert werden:
1) Vinylverbindungen, z.B. Vinylester wie Vinylacetat, Vinyllaurat, Vinylpropionat oder Vinylester der Neononansäure, oder aromatische Vinylverbindungen wie Styrol-Comonomere, beispielsweise Styrol, alpha-Methylstyrol oder polar funktionalisierte Styrole wie Styrole mit Hydroxyl-, Amino-, Nitril-, Carbonsäure-, Phosphonsäure-, Phosphorsäure-, Nitro- oder Sulfonsäuregruppen und deren Salze, wobei die Styrole bevorzugt in para-Position polar funktionalisiert sind.
2) Ungesättigte Di- oder Polycarbonsäuren, z.B. Maleinsäureester wie Dibutylmaleinat oder Dioctylmaleinat, als Salze von Allylverbindungen z.B. Natriumallylsulfonat, als Salze von Amylderivaten z.B. Natriumamylsulfonat.
3) (Meth)Acrylat-Comonomere, dies sind Ester der Acrylsäure und Methacrylsäure, wobei die Estergruppen z.B. gesättigte oder ungesättigte, geradkettige, verzweigte oder cylische Kohlenwasserstoffreste sind, welche eines oder mehrere Heteroatome wie N, O, S, P, F, Cl, Br, I enthalten können. Beispiele solcher Kohlenwasserstoffreste sind geradkettiges, verzweigtes oder cylisches Alkyl, geradkettiges, verzweigtes oder cylisches Alkenyl, Aryl wie Phenyl oder Heterocylyl wie Tetrahydrofurfuryl.

Als (Meth)Acrylat-Comonomere, vorzugsweise für AMPS, kommen beispielsweise in Frage:
a) Acrylsäure, C₁-C₁₄-Alkyl-Acrylsäure wie Methacrylsäure,
b) (Meth)Acrylamide wie Acrylamid, Methacrylamid, Diaceton-Acrylamid, Diaceton-Methacrylamid, N-Butoxymethyl-Acrylamid, N-iso-Butoxymethyl-Acrylamid, N-Butoxymethyl-Methacrylamid, N-iso-Butoxymethyl-Methacrylamid, N-Methylol-Acrylamid, N-Methylol-Methacrylamid;
c) Heterocyclyl-(Meth)Acrylate wie Tetrahydrofurfuryl-acrylat und Tetrahydrofurfuryl-methacrylat oder carbocyclische (Meth)Acrylate wie Isobornyl-acrylat und Isobornyl-methacrylat,
d) Urethan(Meth)Acrylate wie Diurethandiacrylat und Diurathanmethacrylat (CAS: 72869-86-4)
e) C₁-C₁₄-Alkylacrylate wie Methyl-, Ethyl-, n-Propyl-, iso-Propyl-, n-Butyl-, sec. Butyl-, iso-Butyl-, tert Butyl-, n-Pentyl-, iso-Pentyl-, Hexyl- (z.B. n-Hexyl, iso-Hexyl oder Cyclohexyl), Heptyl-, Octyl-(z.B. 2-Ethylhexyl), Nonyl-, Decyl- (z.B. 2-Propylheptyl oder iso-Decyl), Undecyl-, Dodecyl-, Tridecyl- (z.B. iso-Tridecyl), und Tetradecyl-Acrylat; die Alkylgruppen können optional mit einem oder mehreren Halogenatomen (z.B. Fluor, Chlor, Brom oder Iod) substituiert sein, z.B. Trifluoroethyl-Acrylat, oder mit einer oder mehreren Aminogruppen, z.B. Diethylaminoethyl-Acrylat, oder mit einer oder mehreren Alkoxygruppen wie Methoxypropyl-Acrylat, oder mit einer oder mehreren Aryloxygruppen wie Phenoxyethyl-Acrylat.
f) C₂-C₁₄-Alkenylacrylate wie Ethenyl-, n-Propenyl-, iso-Propenyl-, n-Butenyl-, sec. Butenyl-, iso-Butenyl-, tert Butenyl-, n-Pentenyl-, iso-Pentenyl-, Hexenyl- (z.B. n-Hexenyl, iso-Hexenyl oder Cyc-Iohexenyl), Heptenyl-, Octenyl- (z.B. 2-Ethylhexenyl), Nonenyl-, Decenyl- (z.B. 2-Propenylheptyl oder iso-Decenyl), Undecenyl-, Dodecenyl-, Tridecenyl- (z.B. iso-Tridecenyl), und Tetradecenyl-Acrylat, und deren Epoxide wie Glycidyl-Acrylat oder Aziridine wie Aziridin-Acrylat.
g) C₁-C₁₄-Hydroxyalkylacrylate wie Hydroxymethyl-, Hydroxyethyl-, Hydroxy-n-Propyl-, Hydroxy-iso-Propyl-, Hydroxy-n-Butyl-, Hydroxy-sec.-Butyl-, Hydroxy-iso-Butyl-, Hydroxy-tert-Butyl-, Hydroxy-n-Pentyl-, Hydroxy-iso-Pentyl-, Hydroxyhexyl- (z.B. Hydroxy-n-Hexyl, Hydroxy-iso-Hexyl oder Hydroxy-Cyclohexyl), Hydroxyheptyl-, Hydroxyoctyl- (z.B. 2-Ethylhexyl), Hydroxynonyl-, Hydroxydecyl- (z.B. Hydroxy-2-Propylheptyl oder Hydroxy-iso-Decyl), Hydroxyundecyl-, Hydroxydodecyl-, Hydroxytridecyl- (z.B. Hydroxy-iso-Tridecyl), und Hydroxytetradecyl-Acrylat, wobei sich die Hydroxy-Gruppe bevorzugt in endständiger Position (ω-Position) (z.B. 4-Hydroxy-n-butylacrylat) oder in (ω-1)-Position (z.B. 2-Hydroxy-n-propylacrylat) des Alkylrests befindet;
h) Alkylenglykolacrylate, die eine oder mehrere Alkylenglykol-Einheiten enthalten. Beispiele sind i) Monoalkylenglykolacrylate, wie Acrylate von Ethylenglykol, Propylenglykol (z.B. 1,2- oder 1,3-Propandiol), Butylenglykol (z.B. 1,2-, 1,3- oder 1,4-Butandiol, Pentylenglykol (z.B. 1,5-Pentandiol) oder Hexylenglykol (z.B. 1,6-Hexandiol), bei denen die zweite Hydroxylgruppe verethert oder verestert ist, z.B. durch Schwefelsäure, Phosphorsäure, Acrylsäure oder Methacrylsäure, oder ii) Polyalkylenglykolacrylate wie Polyethylenglykolacrylate, Polypropylenglykolacrylate, Polybutylenglykolacrylate, Polypentylenglykolacrylate oder Polyhexylenglykolacrylate, deren zweite Hydroxylgruppe optional verethert oder verestert sein kann, z.B. durch Schwefelsäure, Phosphorsäure, Acrylsäure oder Methacrylsäure;
   Beispiele von (Poly)Alkylenglykol-Einheiten mit veretherten Hydroxylgruppen sind C₁-C₁₄-Alkyloxy-(poly)alkylenglykole (z.B. C₁-C₁₄-Alkyloxy-(poly)alkylenglykolacrylate), Beispiele von (Poly)Alkylenglykol-Einheiten mit veresterten Hydroxylgruppen sind Sulfonium-(poly)alkylenglykole (z.B. Sulfonium-(poly)alkylenglykolacrylate) und deren Salze, (Poly)Alkylenglykoldiacrylate wie 1,4-Butandioldi-acrylat oder 1,6-Hexandioldiacrylat oder (Poly)Alkylenglykolmethacrylatacrylate wie 1,4-Butandiolmeth-acrylatacrylat oder 1,6-Hexandiolmethacrylatacrylat;
   Die Polyalkylenglykolacrylate können eine Acrylatgruppe tragen (z.B. Polyethylenglykolmonoacrylat, Polypropylenglykolmonoacrylat, Polybutylenglykol-monoacrylat, Polypentylenglykolmonoacrylat oder Polyhexylenglykolmonoacrylat) oder zwei oder mehr, bevorzugt zwei, Acrylatgruppen tragen wie Polyethylenglykoldiacrylat, Polypropylenglykoldiacrylat, Polybutylenglykoldiacrylat, Polypentylenglykoldiacrylat oder Polyhexylenglykoldiacrylat;
   Die Polyalkylenglykolacrylate können auch zwei oder mehr voneinander verschiedene Polyalkylenglykol-Blöcke enthalten, z.B. Blöcke von Polymethylenglykol und Polyethylenglykol oder Blöcke von Polyethylenglykol und Polypropylenglykol;
   Der Polymerisationsgrad der Polyalkylenglykol-Einheiten oder Polyalkylenglykol-Blöcke liegt im Allgemeinen im Bereich von 1 bis 20, vorzugsweise im Bereich von 3 bis 10, besonderes bevorzugt im Bereich von 3 bis 6.
i) C₁-C₁₄-Alkylmethacrylate wie Methyl-, Ethyl-, n-Propyl-, iso-Propyl-, n-Butyl-, sec. Butyl-, iso-Butyl-, tert-Butyl-, n-Pentyl-, iso-Pentyl-, Hexyl- (z.B. n-Hexyl, iso-Hexyl oder Cyclohexyl), Heptyl-, Octyl- (z.B. 2-Ethylhexyl), Nonyl-, Decyl- (z.B. 2-Propylheptyl oder iso-Decyl), Undecyl-, Dodecyl-, Tridecyl- (z.B. iso-Tridecyl), und Tetradecyl-Methacrylat; die Alkylgruppen können optional mit einem oder mehreren Halogenatomen (z.B. Fluor, Chlor, Brom oder Iod) substituiert sein, z.B. Trifluoroethyl-Methacrylat, oder mit einer oder mehreren Aminogruppen, z.B. Diethylaminoethyl-Methacrylat, oder mit einer oder mehreren Alkoxygruppen wie Methoxypropyl-Methacrylat, oder mit einer oder mehreren Aryloxygruppen wie Phenoxyethyl-Methacrylat.
j) C₂-C₁₄-Alkenylmethacrylate wie Ethenyl-, n-Propenyl-, iso-Propenyl-, n-Butenyl-, sec. Butenyl-, iso-Butenyl-, tert- Butenyl-, n-Pentenyl-, iso-Pentenyl-, Hexenyl- (z.B. n-Hexenyl, iso-Hexenyl oder Cyclohexenyl), Heptenyl-, Octenyl- (z.B. 2-Ethylhexenyl), Nonenyl-, Decenyl- (z.B. 2-Propenylheptyl oder iso-Decenyl), Undecenyl-, Dodecenyl-, Tridecenyl- (z.B. iso-Tridecenyl), und Tetradecenyl-Methacrylat, und deren Epoxide wie Glycidyl-Methacrylat oder Aziridine wie Aziridin-Methacrylat.
k) C₁-C₁₄-Hydroxyalkylmethacrylate wie Hydroxymethyl-, Hydroxyethyl-, Hydroxy-n-Propyl-, Hydroxy-iso-Propyl-, Hydroxy-n-Butyl-, Hydroxy-sec.-Butyl-, Hydroxy-iso-Butyl-, Hydroxy-tert-Butyl-, Hydroxy-n-Pentyl-, Hydroxy-iso-Pentyl-, Hydroxyhexyl- (z.B. Hydroxy-n-Hexyl, Hydroxy-iso-Hexyl oder Hydroxy-Cyclohexyl), Hydroxyheptyl-, Hydroxyoctyl- (z.B. 2-Ethylhexyl), Hydroxynonyl-, Hydroxydecyl- (z.B. Hydroxy-2-Propylheptyl oder Hydroxy-iso-Decyl), Hydroxyundecyl-, Hydroxydodecyl-, Hydroxytridecyl- (z.B. Hydroxy-iso-Tridecyl), und Hydroxytetradecyl-Methacrylat, wobei sich die Hydroxy-Gruppe bevorzugt in endständiger Position (ω-Position) (z.B. 4-Hydroxy-n-butylmeth acrylat) oder in (ω-1)-Position (z.B. 2-Hydroxy-n-propylmethacrylat) des Alkylrests befindet;
l) Alkylenglykolmethacrylate, die eine oder mehrere Alkylenglykol-Einheiten enthalten. Beispiele sind I) Monoalkylenglykolmethacrylate, wie Methacrylate von Ethylenglykol, Propylenglykol (z.B. 1,2- oder 1,3-Propandiol), Butylenglykol (z.B. 1,2-, 1,3- oder 1,4-Butandiol, Pentylenglykol (z.B. 1,5-Pentandiol) oder Hexylenglykol (z.B. 1,6-Hexandiol), bei denen die zweite Hydroxylgruppe verethert oder verestert ist, z.B. durch Schwefelsäure, Phosphorsäure, Acrylsäure oder Methacrylsäure, oder ii) Polyalkylengly-kolmethacrylate wie Polyethylenglykolmethacrylate, Polypropylenglykol-methacrylate, Polybutylenglykolmethacrylate, Polypentylenglykolmethacrylate oder Polyhexylenglykolmethacrylate, deren zweite Hydroxylgruppe optional verethert oder verestert sein kann, z.B. durch Schwefelsäure, Phosphorsäure, Acrylsäure oder Methacrylsäure;
   Beispiele von (Poly)Alkylenglykol-Einheiten mit veretherten Hydroxygruppen sind C₁-C₁₄-Alkyloxy-(poly)alkylenglykole (z.B. C₁-C₁₄-Alkyloxy-(poly)alkylenglykolmeth-acrylate), Beispiele von (Poly)Alky-lenglykol-Einheiten mit veresterten Hydroxylgruppen sind Sulfonium-(poly)alkylenglykole (z.B. Sulfonium-(poly)alkylenglykolmethacrylate) und deren Salze oder (Poly)Alkylenglykoldimethacrylate wie 1,4-Butandioldimethacrylat;
   Die Polyalkylenglykolmethacrylate können eine Methacrylatgruppe tragen (z.B. Polyethylenglykolmonomethacrylat, Polypropylen-glykolmonomethacrylat, Polybutylenglykol-monomethacrylat, Polypentylenglykolmono-methacrylat oder Polyhexylenglykolmonomethacrylat) zwei oder mehr, bevorzugt zwei, Methacrylatgruppen tragen, wie Polyethylenglykoldimethacrylat, Polypropylenglykoldimethacrylat, Polybutylenglykoldimethacrylat, Polypentylenglykoldi-methacrylat oder Polyhexylenglykoldimethacrylat;
   Die Polyalkylenglykolmethacrylate können auch zwei oder mehr voneinander verschiedene Polyalkylenglykol-Blöcke enthalten, z.B. Blöcke von Polymethylenglykol und Polyethylenglykol oder Blöcke von Polyethylenglykol und Polypropylenglykol (z.B. Bisomer PEM63PHD (Cognis), CAS 58916-75-9);
   Der Polymerisationsgrad der Polyalkylenglykol-Einheiten oder Polyalkylenglykol-Blöcke liegt im Allgemeinen im Bereich von 1 bis 20, vorzugsweise im Bereich von 3 bis 10, besonderes bevorzugt im Bereich von 3 bis 6.

Beispiele bevorzugter (Meth)Acrylat-Comonomere sind 4-Hydroxybutylacrylat, 2-Hydroxypropylmethacrylat, Ammoniumsulfatoethylmethacrylat, Pentapropylenglykolmethacrylat, Acrylsäure, Hexaethylenglykolmethyacrylat, Hexapropylenglykolacrylat, Hexaethylenglykolacrylat, Hydroxyethylmethacrylat, Polyalkylenglycolmethacrylat (CAS-Nr. 589-75-9), Bisomer PEM63PHD, Methoxypolyethylenglycolmethacrylat, 2-Propylheptylacrylat (2-PHA), 1,3-Butandioldimethacrylat (BDDMA), Triethylenglykoldimethacrylat (TEGDMA), Hydroxyethylacrylat (HEA), 2-Hydroxypropylacrylat (HPA), Ethylenglykoldimethacrylat (EGDMA), Glycidylmethacrylat (GMA) und/oder Allylmethacrylat (ALMA).

Die AMPS-Copolymere weisen im Allgemeinen einen Anteil an AMPS-Einheiten von größer 50 Mol-% auf, vorzugsweise im Bereich von 60 bis 95 Mol-%, besonders bevorzugt von 80 bis 99 Mol-%, der Anteil an Comonomeren liegt im Allgemeinen kleiner 50 Mol-%, vorzugsweise im Bereich von 5 bis 40 Mol-%, besonders bevorzugt von 1 bis 20 Mol-%.

Die Copolymere können durch an sich bekannte Verfahren erhalten werden, beispielsweise im Batch- oder im Semibatch-Verfahren. Beispielsweise werden zunächst entsprechende Mengen Wasser und Monomeren in einen temperierbaren Reaktor geleitet und unter Inertgas-Atmosphäre gesetzt. Die Vorlage dann gerührt, auf Reaktionstemperatur gebracht (vorzugsweise im Bereich von ca. 70 bis 80°C) und Initiator zugesetzt, vorzugsweise in Form einer wässrigen Lösung. Als Initiator eignen sich bekannte Initiatoren für radikalische Polymerisationen, beispielsweise Natrium- , Kalium- oder Ammoniumperoxodisulfat, oder H₂O₂ basierte Mischungen, z.B. Mischungen von H₂O₂ mit Zitronensäure. Die maximale Temperatur wird abgewartet und sobald die Temperatur im Reaktor sinkt erfolgt entweder a) die Zudosierung der restlichen Monomere und anschließend eine Nachreaktion (Semibatch-Verfahren), oder b) direkt die Nachreaktion (Batch-Verfahren). Danach wird das erhaltene Reaktionsgemisch auf Raumtemperatur abgekühlt und das Copolymer aus der wässrigen Lösung isoliert, z.B. durch Extraktion mit organischen Lösungsmitteln wie Hexan oder Methylenchlorid und anschließendes Abdestillieren des Lösungsmittels. Danach kann das Copolymer mit organischem Lösungsmittel gewaschen und getrocknet werden. Das erhaltene Reaktionsgemisch kann auch direkt weiterverarbeitet werden, in diesem Falle ist es von Vorteil, der wässrigen Copolymer-Lösung ein Konservierungsmittel zuzusetzen.

Die AMPS-Copolymere eignen sich als Schutzkolloide bei der Herstellung der gemäß der vorliegenden Erfindung einsetzbaren Mikrokapseln.

Die Herstellung der in den Mittel zum Waschen, Reinigen, Konditionieren, Pflegen und/oder Färben von harten oder weichen Oberflächen enthaltenen Mikrokapseln oder Mikrokapseldispersionen erfolgt vorzugsweise, indem der mindestens eine erfindungsgemäß umzusetzende aromatische Alkohol, ausgewählt aus Resorcin und/oder Phloroglucin, und die mindestens eine erfindungsgemäß umzusetzende aldehydische Komponente, die mindestens zwei C-Atome pro Molekül aufweist, in Gegenwart mindestens eines (Meth)Acrylatpolymers, gegebenenfalls in Gegenwart mindestens einer zu verkapselnde Substanz (Kernmaterial), zusammen- und zur Reaktion gebracht werden und durch spätere Temperaturerhöhung die Aushärtung der Kapseln erfolgt. Dabei ist es besonders bevorzugt, dass der pH-Wert im Laufe des Verfahrens erhöht wird.

Vorzugweise wird im Rahmen eines solchen Verfahrens zunächst
a) der mindestens eine aromatische Alkohol und/oder dessen Derivat oder Ether und die mindestens eine aldehydische Komponente und mindestens ein (Meth)Acrylatpolymer und mindestens eine zu verkapselnde Substanz bei einer Temperatur von 40 bis 65°C und einem pH-Wert zwischen 6 und 9, vorzugsweise 7 und 8,5 zusammengebracht und
b) in einem späteren Verfahrensschritt bei einer Temperatur von 40 bis 65°C der pH-Wert auf über 9, vorzugsweise zwischen 9,5 und 11 angehoben, wobei
c) später die Aushärtung der Kapseln durch Temperaturerhöhung auf 60 bis 110°C, vorzugsweise 70 bis 90°C, insbesondere 80°C durchgeführt wird.

Wird jedoch Phloroglucin als Alkoholkomponente verwendet, wird vorteilhafter im Sauren gehärtet; bevorzugt liegt der pH Wert dann bei höchstens 4, insbesondere bevorzugt zwischen 3 und 4, zum Beispiel zwischen 3,2 und 3,5.

Die erzeugbaren Kapseln sind formaldehydfrei und lassen sich als stabile Kern/Schale - Mikrokapseln aus der wässrigen Slurry heraus ohne Probleme zu einem trockenen fließfähigen Pulver verarbeiten.

Die Beladung der Kapseln kann allgemein mit gasförmigen, flüssigen sowie festen Stoffen erfolgen. Bevorzugt werden hydrophobe Materialien eingesetzt. Besonders bevorzugt sind jedoch flüssige Stoffe, Aktivstoffe, insbesondere Riechstoffe und Pflanzenextrakte, sowie Tenside, insbesondere Niotenside, Silikonöle, Paraffine, flüssige nicht-pharmazeutische Additive oder Wirkstoffe, z.B. Öle wie beispielsweise Mandelöl sowie Mischungen vorgenannter. Es ist allerdings am meisten bevorzugt, dass Riechstoffe (Parfümöle) und/ oder Pflanzenextrakte in den Mikrokapseln enthalten sind.

Als Duftstoffe bzw. Riechstoffe bzw. Parfümöle können alle dafür bekannten Stoffe und Gemische eingesetzt werden. Im Sinne dieser Erfindung werden die Begriffe "Riechstoff(e)", "Duftstoffe" und "Parfümöl(e)" synonym gebraucht. Damit sind insbesondere all jene Stoffe oder deren Gemische gemeint, die von Mensch und Tier als Geruch empfunden werden, insbesondere vom Mensch als Wohlgeruch empfunden werden.

Als Duftkomponenten können Parfüme, Parfümöle oder Parfümölbestandteile eingesetzt werden. Parfümöle bzw. Duftstoffe können erfindungsgemäß einzelne Riechstoffverbindungen, z.B. die synthetischen Produkte vom Typ der Ester, Ether, Aldehyde, Ketone, Alkohole und Kohlenwasserstoffe sein. Riechstoffverbindungen vom Typ der Ester sind z.B. Benzylacetat, Phenoxyethylisobutyrat, p-tert-Butylcyclohexylacetat, Linalylacetat, Dimethylbenzylcarbinylacetat (DMBCA), Phenylethylacetat, Benzylacetat, Ethylmethylphenylglycinat, Allylcyclohexylpropionat, Styrallylpropionat, Benzylsalicylat, Cyclohexylsalicylat, Floramat, Melusat und Jasmecyclat. Zu den Ethern zählen beispielsweise Benzylethylether und Ambroxan, zu den Aldehyden z.B. die linearen Alkanale mit 8 bis 18 C-Atomen, Citral, Citronellal, Citronellyloxyacetaldehyd, Cyclamenaldehyd, Lilial und Bourgeonal, zu den Ketonen z.B. die Jonone, alpha-Isomethylionon und Methylcedrylketon, zu den Alkoholen Anethol, Citronellol, Eugenol, Geraniol, Linalool, Phenylethylalkohol und Terpineol, zu den Kohlenwasserstoffen gehören hauptsächlich die Terpene wie Limonen und Pinen. Bevorzugt werden jedoch Mischungen verschiedener Riechstoffe verwendet, die gemeinsam eine ansprechende Duftnote erzeugen.

Solche Parfümöle können auch natürliche Riechstoffgemische enthalten, wie sie aus pflanzlichen Quellen zugänglich sind, z.B. Pine-, Citrus-, Jasmin-, Patchouly-, Rosen- oder Ylang-Ylang-Öl. Ebenfalls geeignet sind Muskateller-Salbeiöl, Kamillenöl, Nelkenöl, Melissenöl, Minzöl, Zimtblätteröl, Lindenblütenöl, Wacholderbeeröl, Vetiveröl, Olibanumöl, Galbanumöl und Labdanumöl sowie Orangenblütenöl, Neroliöl, Orangenschalenöl und Sandelholzöl.

Um wahrnehmbar zu sein, muss ein Riechstoff flüchtig sein, wobei neben der Natur der funktionellen Gruppen und der Struktur der chemischen Verbindung auch die Molmasse eine wichtige Rolle spielt. So besitzen die meisten Riechstoffe Molmassen bis etwa 200 Dalton, während Molmassen von 300 Dalton und darüber eher eine Ausnahme darstellen. Aufgrund der unterschiedlichen Flüchtigkeit von Riechstoffen verändert sich der Geruch eines aus mehreren Riechstoffen zusammengesetzten Parfüms bzw. Duftstoffs während des Verdampfens, wobei man die Geruchseindrücke in "Kopfnote" (top note), "Herz- bzw. Mittelnote" (middle note bzw. body) sowie "Basisnote" (end note bzw. dry out) unterteilt. Da die Geruchswahrnehmung zu einem großen Teil auch auf der Geruchsintensität beruht, besteht die Kopfnote eines Parfüms bzw. Duftstoffs nicht allein aus leichtflüchtigen Verbindungen, während die Basisnote zum größten Teil aus weniger flüchtigen, d. h. haftfesten Riechstoffen besteht. Bei der Komposition von Parfüms können leichter flüchtige Riechstoffe beispielsweise an bestimmte Fixative gebunden werden, wodurch ihr zu schnelles Verdampfen verhindert wird. Bei der nachfolgenden Einteilung der Riechstoffe in "leichter flüchtige" bzw. "haftfeste" Riechstoffe ist also über den Geruchseindruck und darüber, ob der entsprechende Riechstoff als Kopf- oder Herznote wahrgenommen wird, nichts ausgesagt. Haftfeste Riechstoffe, die im Rahmen der vorliegenden Erfindung einsetzbar sind, sind beispielsweise die ätherischen Öle wie Angelikawurzelöl, Anisöl, Arnikablütenöl, Basilikumöl, Bayöl, Bergamottöl, Champacablütenöl, Edeltannenöl, Edeltannenzapfenöl, Elemiöl, Eukalyptusöl, Fenchelöl, Fichtennadelöl, Galbanumöl, Geraniumöl, Gingergrasöl, Guajakholzöl, Gurjunbalsamöl, Helichrysumöl, Ho-Öl, Ingweröl, Irisöl, Kajeputöl, Kalmusöl, Kamillenöl, Kampferöl, Kanagaöl, Kardamomenöl, Kassiaöl, Kiefernnadelöl, Kopalvabalsamöl, Korianderöl, Krauseminzeöl, Kümmelöl, Kuminöl, Lavendelöl, Lemongrasöl, Limetteöl, Mandarinenöl, Melissenöl, Moschuskörneröl, Myrrhenöl, Nelkenöl, Neroliöl, Niaouliöl, Olibanumöl, Orangenöl, Origanumöl, Palmarosaöl, Patschuliöl, Perubalsamöl, Petitgrainöl, Pfefferöl, Pfefferminzöl, Pimentöl, Pine-Öl, Rosenöl, Rosmarinöl, Sandelholzöl, Sellerieöl, Spiköl, Sternanisöl, Terpentinöl, Thujaöl, Thymianöl, Verbenaöl, Vetiveröl, Wacholderbeeröl, Wermutöl, Wintergrünöl, Ylang-Ylang-Öl, Ysop-Öl, Zimtöl, Zimtblätteröl, Zitronellöl, Zitronenöl sowie Zypressenöl. Aber auch die höhersiedenden bzw. festen Riechstoffe natürlichen oder synthetischen Ursprungs können im Rahmen der vorliegenden Erfindung als haftfeste Riechstoffe bzw. Riechstoffgemische, also Duftstoffe, eingesetzt werden. Zu diesen Verbindungen zählen die nachfolgend genannten Verbindungen sowie Mischungen aus diesen: Ambrettolid, α-Amylzimtaldehyd, Anethol, Anisaldehyd, Anisalkohol, Anisol, Anthranilsäuremethylester, Acetophenon, Benzylaceton, Benzaldehyd, Benzoesäureethylester, Benzophenon, Benzylakohol, Benzylacetat, Benzylbenzoat, Benzylformiat, Benzylvalerianat, Borneol, Bornylacetat, α-Bromstyrol, n-Decylaldehyd, n-Dodecylaldehyd, Eugenol, Eugenolmethylether, Eukalyptol, Farnesol, Fenchon, Fenchylacetat, Geranylacetat, Geranylformiat, Heliotropin, Heptincarbonsäuremethylester, Heptaldehyd, Hydrochinon-Dimethylether, Hydroxyzimtaldehyd, Hydroxyzimtalkohol, Indol, Iron, Isoeugenol, Isoeugenolmethylether, Isosafrol, Jasmon, Kampfer, Karvakrol, Karvon, p-Kresolmethylether, Cumarin, p-Methoxyacetophenon, Methyl-n-amylketon, Methylanthranilsäuremethylester, p-Methylacetophenon, Methylchavikol, p-Methylchinolin, Methyl-β-naphthylketon, Methyl-n-nonylacetaldehyd, Methyl-n-nonylketon, Muskon, β-Naphtholethylether, β-Naphtholmethylether, Nerol, Nitrobenzol, n-Nonylaldehyd, Nonylakohol, n-Octylaldehyd, p-Oxy-Acetophenon, Pentadekanolid, β-Phenylethylakohol, Phenylacetaldehyd-Dimethyacetal, Phenylessigsäure, Pulegon, Safrol, Salicylsäureisoamylester, Salicylsäuremethylester, Salicylsäurehexylester, Salicylsäurecyclohexylester, Santalol, Skatol, Terpineol, Thymen, Thymol, γ-Undelacton, Vanillin, Veratrumaldehyd, Zimtaldehyd, Zimatalkohol, Zimtsäure, Zimtsäureethylester, Zimtsäurebenzylester

Zu den leichter flüchtigen Riechstoffen zählen insbesondere die niedriger siedenden Riechstoffe natürlichen oder synthetischen Ursprungs, die allein oder in Mischungen eingesetzt werden können. Beispiele für leichter flüchtige Riechstoffe sind Alkylisothiocyanate (Alkylsenföle), Butandion, Limonen, Linalool, Linaylacetat und -propionat, Menthol, Menthon, Methyl-n-heptenon, Phellandren, Phenylacetaldehyd, Terpinylacetat, Zitral, Zitronellal.

Bevorzugt einsetzbare (insbesondere zu verkapselnde) Riechstoffverbindungen vom Typ der Aldehyde sind Hydroxycitronellal (CAS 107-75-5), Helional (CAS 1205-17-0), Citral (5392-40-5), Bourgeonal (18127-01-0), Triplal (CAS 27939-60-2), Ligustral (CAS 68039-48-5), Vertocitral (CAS 68039-49-6), Florhydral (CAS 125109-85-5), Citronellal (CAS 106-23-0), Citronellyloxyacetaldehyd (CAS 7492-67-3).

Weiterhin ist es bevorzugt, dass das zu verkapselnde Parfüm kein 2-Methyl-Undecanal, Decanal, Benzolacetaldehyd und 3-Phenylprop-2-enal umfasst.

Die Mikrokapseln können vorzugsweise auch ein oder mehrere (vorzugsweise flüssige) hautpflegende und/oder hautschützende Aktivstoffe enthalten. Hautpflegende Aktivstoffe sind alle solche Aktivstoffe, die der Haut einen sensorischen und/oder kosmetischen Vorteil verleihen. Hautpflegende Aktivstoffe sind bevorzugt ausgewählt aus den nachfolgenden Substanzen:
a) Wachse wie beispielsweise Carnauba, Spermaceti, Bienenwachs, Lanolin und/oder Derivate derselben und andere.
b) Hydrophobe Pflanzenextrakte
c) Kohlenwasserstoffe wie beispielsweise Squalene und/oder Squalane
d) Höhere Fettsäuren, vorzugsweise solche mit wenigstens 12 Kohlenstoffatomen, beispielsweise Laurinsäure, Stearinsäure, Behensäure, Myristinsäure, Palmitinsäure, Ölsäure, Linolsäure, Linolensäure, Isostearinsäure und/oder mehrfach ungesättigte Fettsäuren und andere.
e) Höhere Fettalkohole, vorzugsweise solche mit wenigstens 12 Kohlenstoffatomen, beispielsweise Laurylalkohol, Cetylalkohol, Stearylalkohol, Oleylalkohol, Behenylalkohol, Cholesterol und/oder 2-Hexadecanaol und andere.
f) Ester, vorzugsweise solche wie Cetyloctanoate, Lauryllactate, Myristyllactate, Cetyllactate, Isopropylmyristate, Myristylmyristate, Isopropylpalmitate, Isopropyladipate, Butylstearate, Decyloleate, Cholesterolisostearate, Glycerolmonostearate, Glyceroldistearate, Glyceroltristearate, Alkyllactate, Alkylcitrate und/oder Alkyltartrate und andere.
g) Lipide wie beispielsweise Cholesterol, Ceramide und/oder Saccharoseester und andere.
h) Vitamine wie beispielsweise die Vitamine A, C und E, Vitaminalkylester, einschließlich Vitamin-C-Alkylester und andere.
i) Sonnenschutzmittel
j) Phospholipide
k) Derivate von alpha-Hydroxysäuren
l) Germizide für den kosmetischen Gebrauch, sowohl synthetische wie beispielsweise Salicylsäure und/oder andere als auch natürliche wie beispielsweise Neemöl und/oder andere.
m) Silikone
n) Natürliche Öle, z.B. Mandelöl
   sowie Mischungen jeglicher vorgenannter Komponenten.

Bevorzugt enthalten die Mikrokapseln Pflanzenextrakte als Aktivstoff. Üblicherweise werden diese Extrakte durch Extraktion der gesamten Pflanze hergestellt. Es kann aber in einzelnen Fällen auch bevorzugt sein, die Extrakte ausschließlich aus Blüten und/oder Blättern der Pflanze herzustellen. Erfindungsgemäß sind vor allem die Extrakte aus Grünem Tee, Eichenrinde, Brennnessel, Hamamelis, Hopfen, Henna, Kamille, Klettenwurzel, Schachtelhalm, Weißdorn, Lindenblüten, Mandel, Aloe Vera, Fichtennadel, Rosskastanie, Sandelholz, Wacholder, Kokosnuss, Mango, Aprikose, Limone, Weizen, Kiwi, Melone, Orange, Grapefruit, Salbei, Rosmarin, Birke, Malve, Wiesenschaumkraut, Quendel, Schafgarbe, Thymian, Melisse, Hauhechel, Huflattich, Eibisch, Meristem, Ginseng und Ingwerwurzel bevorzugt. Insbesondere bevorzugt sind Extrakte aus Aloe Vera.

Als Extraktionsmittel zur Herstellung der genannten Pflanzenextrakte können Wasser, Alkohole sowie deren Mischungen verwendet werden. Unter den Alkoholen sind dabei niedere Alkohole wie Ethanol und Isopropanol, insbesondere aber mehrwertige Alkohole wie Ethylenglykol und Propylenglykol, sowohl als alleiniges Extraktionsmittel als auch in Mischung mit Wasser, bevorzugt. Pflanzenextrakte auf Basis von Wasser/Propylenglykol im Verhältnis 1:10 bis 10:1 haben sich als besonders geeignet erwiesen.

Die Pflanzenextrakte können erfindungsgemäß sowohl in reiner als auch in verdünnter Form eingesetzt werden. Sofern sie in verdünnter Form eingesetzt werden, enthalten sie üblicherweise ca. 2 bis 80 Gew.-% Aktivsubstanz und als Lösungsmittel das bei ihrer Gewinnung eingesetzte Extraktionsmittel oder Extraktionsmittelgemisch.
Weiterhin kann es bevorzugt sein als Aktivstoff mehrere, insbesondere zwei verschiedene Pflanzenextrakten einzusetzen. Außerdem können Parfümöle und Pflanzenextrakte zusammen als Aktivstoffe eingesetzt werden.

Die Mikrokapseln weisen im Allgemeinen Durchmesser im Bereich von 1 bis 1000 µm auf. Im Rahmen der vorliegenden Erfindung sind vom Begriff Mikrokapsel auch Nanokapseln umfasst, d.h. Kapseln mit einem Durchmesser < 1 µm. Die Kapseln weisen vorzugsweise einen mittleren Durchmesser von 0,1 bis 100 µm auf. Die Wandstärke kann zum Beispiel 0,05 bis 10 µm betragen.

Die beanspruchten Mittel zum Waschen, Reinigen, Konditionieren, Pflegen und/oder Färben von harten oder weichen Oberflächen enthalten neben den beschriebenen Mikrokapseln noch weitere Inhaltstoffe, nämlich zumindest oberflächenaktive Substanzen.

Als oberflächenaktive Substanzen kommen insbesondere anionische Tenside, nichtionische Tenside, kationische, zwitterionische, amphotere Tenside und/oder Emulgatoren in Frage. Besonders bevorzugt ist es jedoch, wenn das Mittel zum Waschen, Reinigen, Konditionieren, Pflegen und/oder Färben von harten oder weichen Oberflächen, anionische, nichtionische und/oder kationische Tenside enthält. Insbesondere der Einsatz einer Mischung aus anionischen und nichtionischen Tensiden ist vorteilhaft. Das Mittel enthält vorzugsweise 0,05 bis 50 Gew.-%, vorteilhafterweise 1 bis 40 Gew.-%, in weiter vorteilhafter Weise 3 bis 30 Gew.-% und insbesondere 5 bis 20 Gew.-% oberflächenaktive Substanz, insbesondere aus den Gruppen der anionischen Tenside, nichtionischen Tenside, kationischen, zwitterionischen, amphotere Tenside und/oder Emulgatoren. Dies entspricht einer bevorzugten Ausführungsform der Erfindung und ermöglicht optimale Reinigungsleistungen.

Besonders bevorzugt ist es, wenn das Mittel zum Waschen, Reinigen, Konditionieren, Pflegen und/oder Färben von harten oder weichen Oberflächen Aniontensid enthält, vorteilhafterweise in Mengen von 0,1 bis 25 Gew. %, in weiter vorteilhafter Weise 1 bis 20 Gew.-%, insbesondere in Mengen von 3 bis 15 Gew.-%, bezogen auf das gesamte Mittel. Dies entspricht einer bevorzugten Ausführungsform der Erfindung und ermöglicht besonders vorteilhafte Reinigungsleistungen. Ein besonders geeignetes Aniontensid ist Alkylbenzolsulfonat, vorzugsweise lineares Alkylbenzolsulfonat (LAS). Wenn das Mittel Alkylbenzolsulfonat enthält, vorteilhafterweise in Mengen von 0,1 bis 25 Gew.-%, in weiter vorteilhafter Weise 1 bis 20 Gew.-%, insbesondere in Mengen von 3 bis 15 Gew.-%, bezogen auf das gesamte Mittel, so liegt eine bevorzugte Ausführungsform der Erfindung vor.

Besonders geeignete Aniontenside sind ferner die Alkylsulfate, insbesondere die Fettalkoholsulfate (FAS), wie z.B. C₁₂-C₁₈-Fettalkoholsulfat. Vorzugsweise können C₈-C₁₈-Alkylsulfate eingesetzt werden, besonders bevorzugt sind C₁₃-Alkylsulfat sowie C₁₃₋₁₅-Alkylsulfat und C₁₃₋₁₇-Alkylsulfat, vorteilhafterweise verzweigtes, insbesondere Alkyl-verzweigtes C₁₃₋₁₇-Alkylsulfat. Besonders geeignete Fettalkoholsulfate leiten sich vom Lauryl- und Myristylalkohol ab, sind also Fettalkoholsulfate mit 12 bzw. 14 Kohlenstoffatomen. Die langkettigen FAS-Typen (C₁₆ bis C₁₈) eignen sich sehr gut zum Waschen bei höheren Temperaturen. Besonders bevorzugt sind Alkylsulfate, die einen niedrigeren Krafft-Punkt aufweisen, vorzugsweise mit Krafft-Punkt kleiner 45, 40, 30 oder 20°C.

Krafft-Punkt ist die Bezeichnung für diejenige Temperatur, bei der die Löslichkeit von Tensiden infolge der Bildung von Micellen stark zunimmt. Der Krafft-Punkt ist ein Tripelpunkt, an dem sich der Festkörper oder hydratisierte Kristalle des Tensids mit dessen gelösten (hydratisierten) Monomeren und Micellen im Gleichgewicht befinden. Bestimmt wird der Krafft-Punkt über eine Trübungsmessung gemäß DIN EN 13955: 2003-03. Wenn das Mittel zum Waschen, Reinigen, Konditionieren, Pflegen und/oder Färben von harten oder weichen Oberflächen Alkylsulfat, insbesondere C₁₂-C₁₈-Fettalkoholsulfat, enthält, vorteilhafterweise in Mengen von 0,1 bis 25 Gew. %, in weiter vorteilhafter Weise 1 bis 20 Gew.-%, insbesondere in Mengen von 3 bis 15 Gew.-%, bezogen auf das gesamte Mittel, so liegt eine bevorzugte Ausführungsform der Erfindung vor.

Andere bevorzugt einsetzbare Aniontenside sind z.B. Alkansulfonate (z.B. sekundäres C13-C18-Alkansulfonat), Methylestersulfonate (z.B. α-C12-C18-Methylestersulfonat) und α-Olefinsulfonate (z.B. α-C14-C18-Olefinsulfonat) und Alkylethersulfate (z.B. C12-C14-Fettalkohol-2EO-ethersulfat) und/oder Seifen. Weitere geeignete Aniontenside werden weiter unten noch beschrieben. Besonders geeignet sind aber FAS und/oder LAS.

Die anionischen Tenside, einschließlich der Seifen, können in Form ihrer Natrium-, Kalium- oder Ammoniumsalze sowie als lösliche Salze organischer Basen, wie Mono-, Di- oder Triethanolamin, vorliegen. Vorzugsweise liegen die anionischen Tenside in Form ihrer Natrium- oder Kaliumsalze, insbesondere in Form der Natriumsalze vor.

Besonders bevorzugt ist es, wenn das Mittel zum Waschen, Reinigen, Konditionieren, Pflegen und/oder Färben von harten oder weichen Oberflächen Niotensid enthält, vorteilhafterweise in Mengen von 0,01 bis 25 Gew.-%, in weiter vorteilhafter Weise 1 bis 20 Gew.-%, insbesondere in Mengen von 3 bis 15 Gew.-%, bezogen auf das gesamte Mittel. Dies entspricht einer bevorzugten Ausführungsform der Erfindung. Besonders bevorzugt ist der Einsatz von Alkylpolyglycolethern, insbesondere in Kombination mit Aniontensid, wie vorzugsweise LAS.

Weitere geeignete Niotenside sind Alkylphenolpolyglycolether (APEO), (ethoxylierte) Sorbitanfettsäure-ester (Sorbitane), Alkylpolyglucoside (APG), Fettsäureglucamide, Fettsäureethoxylate, Aminoxide, Ethylenoxid-Propylenoxid-Blockpolymere, Polyglycerolfettsäureester und/oder Fettsäurealkanolamide. Weitere geeignete Niotenside werden weiter unten noch beschrieben. Niotenside auf Zuckerbasis, wie insbesondere APG, sind besonders bevorzugt.

In einer weiteren bevorzugten Ausführungsform sind die oberflächenaktiven Substanzen Emulgatoren. Emulgatoren bewirken an der Phasengrenzfläche die Ausbildung von wasser- bzw. ölstabilen Adsorptionsschichten, welche die dispergierten Tröpfchen gegen Koaleszenz schützen und damit die Emulsion stabilisieren. Emulgatoren sind daher wie Tenside aus einem hydrophoben und einem hydrophilen Molekülteil aufgebaut. Hydrophile Emulgatoren bilden bevorzugt O/W - Emulsionen und hydrophobe Emulgatoren bilden bevorzugt W/O - Emulsionen. Unter einer Emulsion ist eine tröpfchenförmige Verteilung (Dispersion) einer Flüssigkeit in einer anderen Flüssigkeit unter Aufwand von Energie zur Schaffung von stabilisierenden Phasengrenzflächen mittels Tensiden zu verstehen. Die Auswahl dieser emulgierenden Tenside oder Emulgatoren richtet sich dabei nach den zu dispergierenden Stoffen und der jeweiligen äußeren Phase sowie der Feinteiligkeit der Emulsion. Erfindungsgemäß verwendbare Emulgatoren sind beispielsweise
- Anlagerungsprodukte von 4 bis 30 Mol Ethylenoxid und/oder 0 bis 5 Mol Propylenoxid an lineare Fettalkohole mit 8 bis 22 C-Atomen, an Fettsäuren mit 12 bis 22 C-Atomen und an Alkylphenole mit 8 bis 15 C-Atomen in der Alkylgruppe,
- C12 bis C22-Fettsäuremono- und -diester von Anlagerungsprodukten von 1 bis 30 Mol Ethylenoxid an Polyole mit 3 bis 6 Kohlenstoffatomen, insbesondere an Glycerin,
- Ethylenoxid- und Polyglycerin-Anlagerungsprodukte an Methylglucosid-Fettsäureester, Fettsäurealkanolamide und Fettsäureglucamide,
- C8 bis C22-Alkylmono- und -oligoglycoside und deren ethoxylierte Analoga, wobei Oligomerisierungsgrade von 1,1 bis 5, insbesondere 1,2 bis 2,0, und Glucose als Zuckerkomponente bevorzugt sind,
- Anlagerungsprodukte von 5 bis 60 Mol Ethylenoxid an Rizinusöl und gehärtetes Rizinusöl,
- Partialester von Polyolen mit 3 bis 6 Kohlenstoffatomen mit gesättigten Fettsäuren mit 8 bis 22 C-Atomen,
- Sterine. Als Sterine wird eine Gruppe von Steroiden verstanden, die am C-Atom 3 des Steroid-Gerüstes eine Hydroxylgruppe tragen und sowohl aus tierischem Gewebe (Zoosterine) wie auch aus pflanzlichen Fetten (Phytosterine) isoliert werden. Beispiele für Zoosterine sind das Cholesterin und das Lanosterin. Beispiele geeigneter Phytosterine sind Ergosterin, Stigmasterin und Sitosterin. Auch aus Pilzen und Hefen werden Sterine, die sogenannten Mykosterine, isoliert.
- Phospholipide. Hierunter werden vor allem die Glucose-Phospolipide, die z.B. als Lecithine bzw. Phospahtidylcholine aus z.B. Eidotter oder Pflanzensamen (z.B. Sojabohnen) gewonnen werden, verstanden.
- Fettsäureester von Zuckern und Zuckeralkoholen, wie Sorbit,
- Polyglycerine und Polyglycerinderivate wie beispielsweise Polyglycerinpoly-12-hydroxystearat (Handelsprodukt Dehymuls® PGPH),
- Lineare und verzweigte Fettsäuren mit 8 bis 30 C - Atomen und deren Na-, K-, Ammonium-, Ca-, Mg- und Zn - Salze.

Wenn das Mittel zum Waschen, Reinigen, Konditionieren, Pflegen und/oder Färben von harten oder weichen Oberflächen ein Wasch-, Reinigungs- oder Nachbehandlungsmittel ist, so kann es zusätzlich zu den essentiellen Bestandteilen weitere Inhaltsstoffe enthalten, die die anwendungstechnischen und/oder ästhetischen Eigenschaften des Wasch-, Reinigungs- oder Nachbehandlungsmittels weiter verbessern. Im Rahmen der vorliegenden Erfindung enthält das Wasch-, Reinigungs- oder Nachbehandlungsmittel vorzugsweise zusätzlich einen oder mehrere Stoffe aus der Gruppe der Gerüststoffe, Bleichmittel, Bleichkatalysatoren, Bleichaktivatoren, Enzyme, Elektrolyte, nichtwässrigen Lösungsmittel, pH-Stellmittel, Parfümzusammensetzungen, Parfümträger, Fluoreszenzmittel, Farbstoffe, Hydrotope, Schauminhibitoren, Silikonöle, Soil-Release-Polymere, Vergrauungsinhibitoren, Einlaufverhinderer, Knitterschutzmittel, Farbübertragungsinhibitoren, weitere antimikrobielle Wirkstoffe, Germizide, Fungizide, Antioxidantien, Konservierungsmittel, Korrosionsinhibitoren, Antistatika, Bittermittel, Bügelhilfsmittel, Phobier- und Imprägniermittel, Quell- und Schiebefestmittel, weichmachenden Komponenten sowie UV-Absorber.

Besonders bevorzugte zusätzliche Inhaltsstoffe für Wasch-, Reinigungs- oder Nachbehandlungsmittel sind Gerüststoffe, Enzyme, Elektrolyte, nichtwässrige Lösungsmittel, pH-Stellmittel, Parfümzusammensetzungen, Fluoreszenzmittel, Farbstoffe, Hydrotope, Schauminhibitoren, Soil-Release-Polymere, Vergrauungsinhibitoren, Farbübertragungsinhibitoren, weichmachenden Komponenten, UV-Absorber sowie Mischungen daraus.

In einer bevorzugten Ausführungsform liegen die Wasch-, Reinigungs- oder Nachbehandlungsmittel in flüssiger Form vor und enthalten Wasser als Hauptlösungsmittel.

Die Erfindung betrifft auch die Verwendung eines Wasch-, Reinigungs- oder Nachbehandlungsmittel beim Waschen, Reinigen und/oder Vorbehandeln von textilen Flächengebilden.

Wenn das Mittel zum Waschen, Reinigen, Konditionieren, Pflegen und/oder Färben von harten oder weichen Oberflächen eine kosmetische Zusammensetzung ist, so kann es zusätzlich zu den essentiellen Bestandteilen weitere Inhaltsstoffe enthalten. Bevorzugt enthält die kosmetische Zusammensetzung weiterhin mindestens einen kosmetischen Wirkstoff aus der Gruppe der Oxidationsfarbstoffvorprodukte, der direktziehenden Farbstoffe, der Oxidationsmittel ausgewählt aus Wasserstoffperoxid und dessen Anlagerungsverbindungen an feste Träger, der haarkonditionierenden Wirkstoffe, der desodorierenden und/oder der schweißhemmenden Wirkstoffe, der hautaufhellenden und/oder hautberuhigenden und/oder feuchtlgkeitsspendenden Wirkstoffe, der anorganischen und/oder organischen UV-Filtersubstanzen, der sebumregulierenden Wirkstoffe, der mechanischen Exfoliationsmittel, der antimikrobiellen Wirkstoffe, der haarfestigenden oder Haarstyling-Wirkstoffe, der Antikaries-Wirkstoffe, der Wirkstoffe gegen Zahnsteinbildung und der Mischungen dieser Wirkstoffe. Diese kosmetischen Wirkstoffe sind bevorzugt zu 0,01 bis 70 Gew.-% bezogen auf das Gesamtgewicht des anwendungsbereiten Mittels enthalten.

Ein weiterer Gegenstand der Erfindung ist ein Verfahren zur Herstellung eines flüssigen Mittels zum Waschen, Reinigen, Konditionieren, Pflegen und/oder Färben von harten oder weichen Oberflächen, gekennzeichnet durch das Einrühren einer Mikrokapseldispersion, umfassend mindestens eine erste und eine zweite Mikrokapsel, deren Kapselwände ein Harz umfassen, welches durch Umsetzung
a) mindestens eines aromatischen Alkohols oder dessen Ether oder Derivate mit
b) mindestens einer aldehydischen Komponente, die mindestens zwei C-Atome pro Molekül aufweist, und
c) in Gegenwart mindestens eines (Meth)Acrylat-Polymeren,
   erhältlich ist, und wobei der mindestens eine aromatische Alkohol ausgewählt ist aus Resorcin und/oder Phloroglucin,
in die flüssige Matrix des Mittels zum Waschen, Reinigen, Konditionieren, Pflegen und/oder Färben von harten oder weichen Oberflächen oder durch kontinuierliche Zugabe der genannten Mikrokapseldispersion in eine flüssige Matrix und Vermischen über statische Mischelemente, wobei die Mikrokapseldispersion vorzugsweise zuvor jeweils mit Tensid versetzt wurde, wobei sich die erste und die zweite Mikrokapsel in mindestens einer der umgesetzten Komponenten a) und/oder b) voneinander unterscheiden.

Ein weiterer Gegenstand der Erfindung ist ein Verfahren zur Herstellung eines festen Mittels zum Waschen, Reinigen, Konditionieren, Pflegen und/oder Färben von harten oder weichen Oberflächen, gekennzeichnet
(i) durch das Zumischen einer Mikrokapseldispersion, umfassend mindestens eine erste und eine zweite Mikrokapsel, deren Kapselwände ein Harz umfassen, welches durch Umsetzung
   a) mindestens eines aromatischen Alkohols oder dessen Ether oder Derivate mit
   b) mindestens einer aldehydischen Komponente, die mindestens zwei C-Atome pro Molekül aufweist, und
   c) in Gegenwart mindestens eines (Meth)Acrylat-Polymeren,
      erhältlich ist, und wobei der mindestens eine aromatische Alkohol ausgewählt ist aus Resorcin und/oder Phloroglucin,
   in die übrige Matrix des Mittels zum Waschen, Reinigen, Konditionieren, Pflegen und/oder Färben von harten oder weichen Oberflächen, oder
(ii) durch das Zumischen der genannten Mikrokapseln in granulierter oder geträgerter Form in die übrige Matrix, oder
(iii) durch das Zumischen der genannten Mikrokapseln in getrockneter Form in die übrige Matrix, wobei sich die erste und die zweite Mikrokapsel in mindestens einer der umgesetzten Komponenten a) und/oder b) voneinander unterscheiden.

### Beispiele

### I. Synthesebeispiele:

### Beispiel I.1: Herstellung von Copolymeren

### a) AMPS-Hydroxybutylacrylat

Für den 1500 g Ansatz werden 891 g demineralisiertes Wasser zusammen mit 585 g AMPS (50% wässrige Lösung) und 7,5 g 4-Hydroxybutylacrylat (HBA) in den Reaktor gefüllt und unter Schutzgas-Atmosphäre gesetzt. Das Reaktionsgemisch wird unter Rühren (400 rpm) auf 75°C aufgeheizt. Von dem wasserlöslichen Initiator Natriumperoxodisulfat werden 0,03 g in 15 g Wasser gelöst und mittels einer Spritze in den Reaktor injiziert, wenn die Reaktionstemperatur erreicht ist. Nach Erreichen der Maximaltemperatur beginnt eine Stunde Nachreaktion. Anschließend wird der Ansatz auf Raumtemperatur abgekühlt und mit 1,5 g Konservierungsmittel versetzt.

Die wässrige Lösung wird charakterisiert über die Viskosität, Festkörper-Gehalt und den pH-Wert. Die Viskosität beträgt 540 mPas (gemessen bei 20 rpm Brookfield), der Festkörper-Gehalt beträgt 21% und der pH-Wert liegt bei 3,3. Es werden 3 g Copolymer auf eine Petrischale gegeben und 24 Stunden bei 160°C im Trockenschrank getrocknet. Die Auswaage beträgt 0,69 g, was einer Ausbeute von 21,6% entspricht.

### b) AMPS-Polyalkylenglykolmonomethacrylat

Die Vorlage besteht aus 912 g demineralisiertem Wasser, 240 g AMPS und 7,5 g Poly(ethylen/propylen)glykolmonomethacrylat (Bisomer PEM 63P HD von Cognis, CAS-Nr. 589-75-9). Die Mischung wird unter Schutzgas-Atmosphäre gesetzt. Das Reaktionsgemisch wird unter Rühren (400 rpm) auf 75°C aufgeheizt. 1,5 g Natriumperoxodisulfat werden in 15 g Wasser gelöst und mit einer Spritze in den Reaktor überführt. Nachdem die Temperatur im Reaktor ein Maximum erreicht hat und zu sinken beginnt, werden 240 g AMPS mit 83 g PEM 63P HD mittels Schlauchpumpe über einen Zeitraum von einer Stunde zu dosiert. Anschließend erfolgt eine halbstündige Nachreaktion. Anschließend wird der Ansatz auf Raumtemperatur abgekühlt und mit 1,5 g Konservierungsmittel versetzt.
Die wässrige Lösung wird charakterisiert über die Viskosität, Festkörper-Gehalt und den pH-Wert. Die Viskosität beträgt 110 mPas (gemessen bei 20 rpm Brookfield), der Festkörper-Gehalt beträgt 23% und der pH-Wert ist 3,1. Es werden 3 g Copolymer auf eine Petrischale gegeben und 24 Stunden bei 160°C im Trockenschrank getrocknet. Die Auswaage beträgt 0,68 g, was einer Ausbeute von 21,6% entspricht.

### 1. Beispiel 1.2: Resorcin-Kapsel

In einem 400 ml-Becherglas werden 5,5 g Resorcin unter Rühren (Rührgeschwindigkeit: etwa 1500 U/min) in 70 g Wasser gelöst und anschließend mit 2,0 g Natriumcarbonat-Lösung (20 Gew.-%ig) versetzt, worauf der pH-Wert bei ca. 7,9 liegt. Diese Lösung wird auf eine Temperatur von etwa 52°C erwärmt. Anschließend werden 25,5 g Glutardialdehyd hinzugegeben.

Das Gemisch wird weitere ca. 10 Minuten bei einer Rührgeschwindigkeit von etwa 1500 U/min und einer Temperatur von etwa 52°C gerührt (Vorkondensationszeit). Danach werden etwa 20 g Wasser ergänzt und ca. 2 Minuten später 1 g das Schutzkolloid a) Copolymer I.1a und wieder ca. 2 Minuten später 55 g Butylphenylacetat (CAS-Nummer 122-43-0; Riechstoff mit honigartigem Geruch) hinzugefügt. Direkt im Anschluss wird die Rührgeschwindigkeit auf etwa 4000 U/min erhöht und etwa zur gleichen Zeit erfolgt die Zugabe von 20,0 g Natriumcarbonat-Lösung (20 Gew.-%ig). Danach liegt der pH-Wert der Mischung bei etwa 9,7. In der Folgezeit nehmen die Viskosität und das Volumen der Mischung zu. Es wird so lange bei einer Rührgeschwindigkeit von etwa 4000 U/min weitergerührt, bis die Viskosität wieder gesunken ist. Erst danach wird die Rührgeschwindigkeit auf etwa 1500 U/min abgesenkt. Bei einer Temperatur von etwa 52°C und etwa gleichbleibender Rührgeschwindigkeit wird der Ansatz weitere ca. 60 Minuten gerührt. Diese Phase nennt man auch Ruhephase. Im Anschluss daran wird die Mischung auf ca. 80°C aufgeheizt und die Kapseln bei dieser Temperatur über einen Zeitraum von 3 Stunden ausgehärtet.
Kapselgrößenverteilung - D (90) 5 bis 10 µm; Verkapselungseffizienz ca. 90%; Trocknungsausbeute > 90%; Festkörper der Slurry ca. 40 Gew.-%.

Zusätzlich zu den Butylphenylacetat-haltigen Resorcinmikrokapseln des Beispiels I.2. wurden weitere Mikrokapseln nach analogen Verfahren hergestellt:
- Beispiel I.3.: Hydroxycitronellal-haltige Resorcinmikrokapseln,
- Beispiel I.4.: Helional-haltige Resorcinmikrokapseln,
- Beispiel I.5.: Citral-haltige Resorcinmikrokapseln,
- Beispiel I.6.: Bourgeonal-haltige Resorcinmikrokapseln,
- Beispiel I.7.: Triplal-haltige Resorcinmikrokapseln,
- Beispiel I.8.: Ligustral-haltige Resorcinmikrokapseln,
- Beispiel I.9.: Vertocitral-haltige Resorcinmikrokapseln,
- Beispiel I.10.: Florhydral-haltige Resorcinmikrokapseln,
- Beispiel I.11.: Citronellal-haltige Resorcinmikrokapseln,
- Beispiel I.12.: Citronellyloxyacetaldehyd-haltige Resorcinmikrokapseln,
- Beispiel I.13.: Aloe Vera-Extrakt-haltige Resorcinmikrokapseln.

In einer weiteren Beispielserie wurden Phloroglucin-Mikrokapseln hergestellt. Analog zum Verfahren gemäß Beispiel I.2. wurden die dort eingesetzten 5,5 g Resorcin vollständig durch 6,3 g Pholoroglucin ersetzt. Auf diese Weise erhielt man:
- Beispiel I.14.: Butylphenylacetat-haltige Phloroglucin-Mikrokapseln,
- Beispiel I.15.: Hydroxycitronellal-haltige Phloroglucin-Mikrokapseln,
- Beispiel I.16.: Helional-haltige Phloroglucin-Mikrokapseln,
- Beispiel I.17.: Citral-haltige Phloroglucin-Mikrokapsein,
- Beispiel I.18.: Bourgeonal-haltige Phloroglucin-Mikrokapseln,
- Beispiel I.19.: Triplal-haltige Phloroglucin-Mikrokapseln,
- Beispiel I.20.: Ligustral-haltige Phloroglucin-Mikrokapseln,
- Beispiel I.21.: Vertocitral-haltige Phloroglucin-Mikrokapseln,
- Beispiel I.22.: Florhydral-haltige Phloroglucin-Mikrokapseln,
- Beispiel I.23.: Citronellal-haltige Phloroglucin-Mikrokapseln,
- Beispiel I.24.: Citronellyloxyacetaldehyd-haltige Phloroglucin-Mikrokapseln,
- Beispiel I.25.: Aloe Vera-Extrakt-haltige Phloroglucin-Mikrokapseln.

In den beiden Beispielserien I.3 bis I.13 (Resorcin) bzw. I.14 bis I.25 (Phloroglucin) wurde außerdem bei der Synthese der Mikrokapseln 25,5 g Glutardialdehyd durch 21,9 g Succindialdehyd ersetzt.

### II. Kapselmischungen

Es wurden Mischungen von Resorcin-Mikrokapseln, erhalten durch Umsetzung von Resorcin mit Glutardialdehyd gemäß Beispiel I.2, mit Phloroglucin-Mikrokapseln, erhalten durch Umsetzung von Phloroglucin mit Glutardialdehyd gemäß Beispiel I.2, hergestellt. Alle eingesetzten Mikrokapseln enthielten dieselbe Parfümzusammensetzung. Die Kapselmischungen mit unterschiedlichen Anteilen an Resorcin- und Phloroglucin-Mikrokapseln wurden durch Vermischen der entsprechenden Anteile der jeweiligen Mikrokapseln erhalten (Zusammensetzungen B, C und D). Für Vergleichsexperimente wurden die jeweiligen Kapseln alleine eingesetzt (Zusammensetzungen A und E).

| | |
|---|---|
| Kapselzusammensetzung A: | 100% parfümhaltige Phloroglucin-Mikrokapseln |
| Kapselzusammensetzung B: | 5% parfümhaltige Phloroglucin-Mikrokapseln und 95% parfümhaltige Resorcin-Mikrokapseln |
| Kapselzusammensetzung C: | 10% parfümhaltige Phloroglucin-Mikrokapseln und 90% parfümhaltige Resorcin-Mikrokapseln |
| Kapselzusammensetzung D: | 20% parfümhaltige Phloroglucin-Mikrokapseln und 80% parfümhaltige Resorcin-Mikrokapseln |
| Kapselzusammensetzung E: | 100% parfümhaltige Resorcin-Mikrokapseln |

### III. Anwendungsbeispiele

Alle Mengenangaben beziehen sich, soweit nicht anders vermerkt, auf Gew.-% Aktivstoff.

### Beispiel III.1: Flüssiges Konditioniermittel

| | Gew.-% |
|---|---|
| Esterquat^{[a]} | 15 |
| 2-Propanol | 1,7 |
| Silikonöl | 5 |
| MgCl x 6 H2O | 0,5 |
| Mikrokapseln^{[b]} | 0,2 |
| Wasser, vollentsalzt | ad 100 |

| | |
|---|---|
| ^{[a]} N-Methyl-N(2-hydroxyethyl)-N,N-(ditalgacyloxyethyl)ammonium-methosulfat ^{[b]} Mikrokapsel-Zusammensetzung gemäß Beispiel II. A bis E | |

Die Rezeptur wurde durch Aufschmelzen des Esterquats in Wasser hergestellt. Das aufgeschmolzene Esterquat wurde anschließend mit einem hochdispergierendem Gerät gerührt und die restlichen Komponenten wurden hinzugefügt. Die Parfümzugabe und Zugabe der Mikrokapseln erfolgte bei leichter Rührung nach Abkühlung der Mischung auf unter 30°C.

### Beispiel III.2: Konditioniersubstrat

Zur Herstellung des Konditioniersubstrates wurden Vliese aus Cellulose (Fläche: 24,5 cm x 39 cm) mit 20 g des flüssigen Konditioniermittels gemäß Beispiel III.1 getränkt.

### Beispiel III.3: Flüssiges Reinigungsmittel

| Rohstoff | Menge in Gew.-% |
|---|---|
| C12-18 Fettsäure, Na-Salz | 0,7 |
| C10-13 Alkylbenzolsulfonat Na-Salz | 6,4 |
| Natriumcitrat | 1,5 |
| Ethanol | 2,1 |
| Cumolsulfonat,Na | 1,5 |
| C12-18 Fettalkohol + 7EO | 1,5 |
| C8-Fettalkoholsulfat,Na-Salz | 1,5 |
| Mikrokapseln^{[a]} | 0,5 |
| Parfüm | 0,7 |
| Wasser | ad 100 |

| | |
|---|---|
| ^{[a]} Mikrokapsel-Zusammensetzung gemäß Beispiel II. A bis E. | |

### Beispiel III.5: Flüssigwaschmittel

| Rohstoff | Menge in Gew.-% |
|---|---|
| C12-14 Fettsäure | 8,8 |
| C12-18 Fettalkohol + 7EO | 24,0 |
| Alkylpolyglucosid | 2,0 |
| C12-14-2EO -sulfat | 5,0 |
| C16-18 Fettsäure | 6,8 |
| NaOH 50% | 3,0 |
| Citronensäure x 1H2O | 1,0 |
| Glycerin 99,5% | 7,5 |
| Ethanol | 1,0 |
| Silikonöl | 0,3 |
| Polyvinylpyrrolidon | 0,5 |
| HEDP-4Na | 0,5 |
| Enzym, Farbstoff, Parfum, | 0,8 |
| Mikrokapseln^{[a]} | 0,7 |
| Wasser | ad 100 |

| | |
|---|---|
| ^{[a]} Mikrokapsel-Zusammensetzung gemäß Beispiel II. A bis E. | |

### Beispiel III.6: Festes Waschmittel

| Rohstoff | Menge in Gew.-% |
|---|---|
| Alkylbenzolsulfonat (Natriumsalz) | 12 |
| Carboxymethylcellulose | 1 |
| Enzyme | 1 |
| Niotensid | 3 |
| (1-Hydroxyethyliden)bisphosphonat | 1 |
| Natriumcarbonat | 25 |
| Natriumpercarbonat | 12 |
| Natriumsulfat | 27 |
| Polyacrylat^{[a]} | 3 |
| Entschäumer | 2 |
| N,N,N',N'-Tetraacetylethylendiamin | 3 |
| Wasser | 3 |
| Parfüm | 0,15 |
| Mikrokapseln^{[b]} | 1,0 |
| Natriumsilicat^{[c]} | ad 100 |
| Summe | 100 |

| | |
|---|---|
| ^{[a]}Polyacrylat: Polyacrylsäure, Natriumsalz; M = 4500 g/mol ^{[b]} Mikrokapsel-Zusammensetzung gemäß Beispiel II. A bis E. ^{[c]}Natriumsilicat: amorphes Natriumsilicat mit Na₂O:SiO₂ = 2,4 | |

### Beispiel III.7: Waschmittel-Gel

| Rohstoff | Menge in Gew.-% |
|---|---|
| Alkylpolyglucosid | 2,00 |
| C12-14 -Fettsäure | 8,80 |
| C16-18 - Fettsäure | 6,80 |
| NaOH 50% | 3,00 |
| Citronensäure x 1 H₂O | 1,00 |
| Glycerin 99,5% | 7,50 |
| Ethanol | 1,00 |
| Silikonentschäumer | 0,30 |
| Borsäure | 1,00 |
| 1 -Hydroxyethylendiphosphonsäure | 0,50 |
| Vinylimidazol-Vinylpyrrolidon-Copolymer | 1,67 |
| Mikrokapseln^{[a]} | 0,8 |
| Parfüm | 1,3 |
| Wasser | ad 100 |

| | |
|---|---|
| ^{[a]} Mikrokapsel-Zusammensetzung gemäß Beispiel II. A bis E | |

### Beispiel III.8: Bügelwasser

| Rohstoff | Menge in Gew.-% |
|---|---|
| Ethanol | 2 |
| Wasserstoffperoxid | 0,01 |
| Parfüm | 0,05 |
| Mikrokapseln^{[c]} | 0,02 |
| Wasser mit 5° dH | ad 100 Gew.-% |

| | |
|---|---|
| ^{[c]} Mikrokapsel-Zusammensetzung gemäß Beispiel II. A bis E. | |

### Beispiel III.9: Antitranspirant-Wachsstifte

| Rohstoff | Menge in Gew.-% |
|---|---|
| PPG-14 Butylether | 15,00 |
| Hydrogeniertes Castor Oil | 1,00 |
| Stearylalkohol | 20,00 |
| Ceteareth-30 | 3,00 |
| Parfüm | 1,00 |
| Aluminumchlorohydrate | 20,00 |
| Allantoin | 0,10 |
| Cocoglycerides (FP 30 - 32°C) | 4,00 |
| Talk | 3,00 |
| Mikrokapseln^{[a]} | 0,20 |
| Tocopherylacetat | 0,20 |
| Cyclopentasiloxan | ad 100 |

| | |
|---|---|
| ^{[a]} Mikrokapsel-Zusammensetzung gemäß Beispiel II. A bis E. | |

### Beispiel III.10: Suspensionen zum Versprühen als Antitranspirant-Spray

| Rohstoff | Menge in Gew.-% |
|---|---|
| Parfüm | 5,00 |
| Aluminumchlorohydrat (aktiviert) | 30,00 |
| Mikrokapseln^{[a]} | 2,00 |
| Isopropylpalmitat | 5,00 |
| C12-15-Alkylbenzoat | -- |
| Cosmacol PLG^{[b]} | -- |
| Disteardimonium Hectorite | 4,50 |
| Propylencarbonat | 1,50 |
| Ethylhexylpalmitat | ad 100 |

| | |
|---|---|
| ^{[a]} Mikrokapsel-Zusammensetzung gemäß Beispiel II. A bis E. ^{[b]} Cosmacol PLG (INCI: Di-C12-13 Alkyltartrat, tri-C12-13 Alkylcitrat, Silica) | |

### Beipiel IV: Waschtests

Für die Waschtests wurden verschiedene Kapselmischungen aus Beispiel II. A bis E getestet.

Als Prüftextilien wurde 30x30 cm großer Baumwollfrottee eingesetzt. Die Prüftextilien wurden mit weiteren 3 kg Baumwollfrottee und Halbleinen in einer Waschmaschine (Miele Softtronic W1734) bei 40°C mit 60 mL unparfümiertem Universalwaschmittelpulver im Hauptwaschgang bei einer Wasserhärte von 16°dH und einer Schleuderzahl von 1200 U/min gewaschen.

Anschließend erfolgte ein Spülgang mit 40 mL des Weichspülers gemäß Beispiel III.1 der insgesamt 0,20 Gew.-% der jeweiligen Mikrokapseln gemäß Beispiel II. enthielt. Die Prüftextilien wurden bei einer Temperatur von 20°C und einer Luftfeuchtigkeit von 60% rh hängend getrocknet.

Beurteilt wurden der Boosteffekt nach Reibung der trockenen Wäsche sowie die Farbgebung und die Sedimentation bzw. Agglomeratbildung im fertigen Mittel jeweils frisch, nach 4 Wochen Lagerung bei 5°C, nach 4 Wochen Lagerung bei Raumtemperatur (RT) und nach 4 Wochen Lagerung bei 40°C. Die Beurteilung erfolgte durch 8 parfümistisch geschulte Personen und wurde je zweimal reproduziert. Die Ergebnisse sind im Folgenden aufgelistet.

### Ergebnisse des Waschtests

### Beispiel IV.1 Boosteffekt

1 = kein Boost, 2 = leichter Boost, 3 = mittlerer Boost, 4 = starker Boost, 5 = sehr starker Boost

| Kapselzusammensetzung (Beispiel II.) | Boosteffekt nach Reibung (frisch) | Boosteffekt nach Reibung (4 Wochen 5°C) | Boosteffekt nach Reibung (4 Wochen RT) | Boosteffekt nach Reibung (4 Wochen 40°C) |
|---|---|---|---|---|
| A | 5 | 5 | 5 | 4 |
| B | 5 | 5 | 4 | 2 |
| C | 5 | 5 | 4 | 3 |
| D | 5 | 5 | 4 | 3,5 |
| E | 5 | 4,5 | 3,5 | 1,1 |

### Beispiel IV.2 Farbgebung des Mittels

0 = keine Farbveränderung, 1 = akzeptabel, leichte Farbveränderung, 2 = mittlere Farbveränderung, 3 = starke Farbveränderung,

| Kapselzusammensetzung (Beispiel II.) | Farbe (frisch) | Farbe (4 Wochen 5°C) | Farbe (4 Wochen RT) | Farbe (4 Wochen 40°C) |
|---|---|---|---|---|
| A | 2 | 2 | 2,5 | 2,5 |
| B | 0,3 | 0,3 | 0,7 | 0,7 |
| C | 0,5 | 0,5 | 0,9 | 0,9 |
| D | 0,8 | 0,8 | 1 | 1 |
| E | 0,2 | 0,2 | 0,5 | 0,5 |

### Beispiel IV.3 Sedimentation bzw. Agglomeratbildung im Produkt

0 = keine Sedimentation, 1 = akzeptabel, sehr leichte Sedimentation, 2 = leichte Sedimentation, 3 = starke Sedimentation

| Kapselzusammensetzung (Beispiel II.) | Sedimentation (frisch) | Sedimentation (4 Wochen 5°C) | Sedimentation (4 Wochen RT) | Sedimentation (4 Wochen 40°C) |
|---|---|---|---|---|
| A | 2 | 3 | 3 | 3 |
| B | 1 | 1,5 | 1,5 | 1,5 |
| C | 1 | 1,7 | 1,7 | 1,7 |
| D | 1 | 2 | 2 | 2 |
| E | 0,5 | 1,2 | 1,2 | 1,2 |

Wie die Versuche zeigen, tritt bei reinen Phloroglucin-Mikrokapsel (Kapselzusammensetzung A) eine deutliche Sedimentation bei Lagerung auf. Außerdem kommt es bei diesen Kapseln zu mittleren bis starken Farbveränderungen des fertigen Mittels zum Waschen, Reinigen, Konditionieren, Pflegen und/oder Färben von harten oder weichen Oberflächen. Vorteil der Phloroglucin-Mikrokapseln ist jedoch ihr starker bis sehr starker Boost-Effekt auch noch nach vier Wochen. Bei reinen Resorcin-Mikrokapseln (Kapselzusammensetzung E) tritt hingegen keine Farbveränderung und nur sehr leichte Sedimentation auf. Der Boost-Effekt lässt jedoch bei Lagerung sehr stark nach, insbesondere bei 40°C ist nach vier Wochen kein Boost Effekt mehr vorhanden.

Die Tests zeigen deutlich, dass durch eine Kombination von Phloroglucin- mit Resorcin-Mikrokapseln (Kapselzusammensetzungen B, C und D), nur eine leichte Farbveränderung des Endprodukts bei leichter Sedimentation der Kapseln auftritt. Der Boost-Effekt nimmt jedoch überraschender Weise in deutlich höherem Maße zu, als die Anteile an Phloroglucin-Mikrokapseln erwarten lassen. Insbesondere nach Lagerung bei 40°C ist der synergistische Effekt der Kapselzusammensetzungen B, C und D gegenüber den reinen Kapselzusammensetzungen A und E besonders deutlich.

Vergleichbar gute Ergebnisse der Kapselzusammensetzungen B, C und D konnten mit weiteren Wasch-, Reinigungs- oder Nachbehandlungsmitteln (Beispiele III.2 bis III.8) sowie mit kosmetischen Zusammensetzungen (Beispiele III.9 und III.10) erzielt werden.

## Patentansprüche

1. Mittel zum Waschen, Reinigen, Konditionieren, Pflegen und/oder Färben von harten oderweichen Oberflächen, umfassend
i) mindestens eine oberflächenaktive Substanz, sowie
ii) ein Gemisch aus mindestens einer ersten und einer zweiten Mikrokapsel, deren Kapselwände jeweils ein Harz umfassen, welches durch Umsetzung
a) mindestens eines aromatischen Alkohols oder dessen Ether oder Derivate mit
b) mindestens einer aldehydischen Komponente, die mindestens zwei C-Atome pro Molekül aufweist, und
c) in Gegenwart mindestens eines (Meth)Acrylat-Polymeren,
erhältlich ist,
**dadurch gekennzeichnet, dass** sich die erste und die zweite Mikrokapsel in mindestens einer der umgesetzten Komponenten a) und/oder b) voneinander unterscheiden und wobei der mindestens eine aromatische Alkohol ausgewählt ist aus Resorcin und/oder Phloroglucin.

2. Mittel nach Anspruch 1 **dadurch gekennzeichnet, dass** sich die erste und die zweite Mikrokapsel in der umgesetzten Komponenten a) voneinander unterscheiden.

3. Mittel nach Anspruch 1 oder 2 **dadurch gekennzeichnet, dass** sich die erste und die zweite Mikrokapsel in der umgesetzten Komponenten b) voneinander unterscheiden.

4. Mittel nach einem der Ansprüche 1 bis 3, **dadurch gekennzeichnet, dass** die aldehydische Komponente b) ausgewählt ist aus Valeraldehyd, Capronaldehyd, Caprylaldehyd, Decanal, Succindialdehyd, Cyclohexancarbaldehyd, Cyclopentancarbaldehyd, 2-Methyl-1-propanal, 2-Methylpropionaldehyd, Acetaldehyd, Acrolein, Aldosteron, Antimycin A, 8'-Apo-β-caroten-8'-al, Benzaldehyd, Butanal, Chloral, Citral, Citronellal, Crotonaldehyd, Dimethylaminobenzaldehyd, Folinsäure, Fosmidomycin, Furfural, Glutaraldehyd, Glycerinaldehyd, Glykolaldehyd, Glyoxal, Glyoxylsäure, Heptanal, 2-Hydroxybenzaldehyd, 3-Hydroxybutanal, Hydroxymethylfurfural, 4-Hydroxynonenal, Isobutanal, Isobutyraldehyd, Methacrolein, 2-Methylundecanal, Mucochlorsäure, N-Methylformamid, 2-Nitrobenzaldehyd, Nonanal, Octanal, Oleocanthal, Orlistat, Pentanal, Phenylethanal, Phycocyanin, Piperonal, Propanal, Propenal, Protocatechualdehyd, Retinal, Salicylaldehyd, Secologanin, Streptomycin, Strophanthidin, Tylosin, Vanillin, Zimtaldehyd und Mischungen daraus.

5. Mittel nach einem der Ansprüche 1 bis 4 **dadurch gekennzeichnet, dass** die erste Mikrokapsel Phloroglucin und die zweite Mikrokapsel Resorcin als aromatischen Alkohol a) enthält.

6. Mittel nach Anspruch 5 **dadurch gekennzeichnet, dass** die erste und die zweite Mikrokapsel in einem Verhältnis von 1:30 bis 1:3, bevorzugt von 1:19 bis 1:4 und insbesondere bevorzugt von 1:9 bis 1:4 enthält.

7. Mittel nach einem der Ansprüche 1 bis 6, **dadurch gekennzeichnet, dass** es die in Anspruch 1 genannten Mikrokapseln in Mengen von 0,0001 bis 50 Gew.-%, vorzugsweise 0,01 bis 20 Gew.-%, und insbesondere 0,1 bis 5 Gew.-%, enthält, bezogen auf das gesamte Mittel.

8. Mittel nach einem der Ansprüche 1 bis 7, **dadurch gekennzeichnet, dass** in der ersten und/oder zweiten Mikrokapsel Aktivstoffe, bevorzugt Riechstoffe, enthalten sind.

9. Verfahren zur Freisetzung eines Aktivstoffes aus einer Mikrokapsel, welche sich auf einer Oberfläche befindet, wobei die Mikrokapsel durch Kontakt eines Mittels zum Waschen, Reinigen, Konditionieren, Pflegen und/oder Färben von harten oder weichen Oberflächen, umfassend
i) mindestens ein Tensid, sowie
ii) ein Gemisch aus mindestens einer ersten und einer zweiten Mikrokapsel, die jeweils mindestens einen Aktivstoff enthalten und deren Kapselwände ein Harz umfassen, welches durch Umsetzung
a) mindestens eines aromatischen Alkohols oder dessen Ether oder Derivate mit
b) mindestens einer aldehydischen Komponente, die mindestens zwei C-Atome pro Molekül aufweist, und
c) in Gegenwart mindestens eines (Meth)Acrylat-Polymeren,
erhältlich ist,
**dadurch gekennzeichnet, dass** sich die erste und die zweite Mikrokapsel in mindestens einer der umgesetzten Komponenten a) und/oder b) voneinander unterscheiden und wobei der mindestens eine aromatische Alkohol ausgewählt ist aus Resorcin und/oder Phloroglucin, auf die Oberfläche aufgebracht wird und anschließend das Freisetzen des Aktivstoffes durch mechanische Krafteinwirkung erfolgt.

10. Verfahren nach Anspruch 9 **dadurch gekennzeichnet, dass** das Freisetzen des Aktivstoffes durch Reibung erfolgt.

11. Verfahren nach einem der Ansprüche 9 oder 10 wobei es sich bei der Oberfläche um eine textile Oberfläche handelt und das Mittel ein Wasch-, Reinigungs- oder Nachbehandlungsmittel ist.

## Claims

1. An agent for washing, cleaning, conditioning, maintaining and/or coloring hard or soft surfaces, comprising
i) at least one surface-active substance, and
ii) a mixture of at least one first and one second microcapsule of which the capsule walls each comprise a resin that can be obtained by reacting
a) at least one aromatic alcohol or an ether or derivatives thereof with
b) at least one aldehydic component that comprises at least two C atoms per molecule
c) in the presence of at least one (meth)acrylate polymer,
**characterized in that** the first and the second microcapsule differ from each other in at least one of the reacted components a) and/or b) and wherein the at least one aromatic alcohol is selected from resorcinol and/or phloroglucinol.

2. The agent according to claim 1, **characterized in that** the first and the second microcapsule differ from each other in the reacted components a).

3. The agent according to claim 1 or 2, **characterized in that** the first and the second microcapsule differ from each other in the reacted components b).

4. The agent according to one of claims 1 to 3, **characterized in that** the aldehydic component b) is selected from valeraldehyde, caproaldehyde, capryl aldehyde, decanal, succindialdehyde, cyclohexane carbaldehyde, cyclopentane carbaldehyde, 2-methyl-1-propanal, 2-methyl propionaldehyde, acetaldehyde, acrolein, aldosterone, antimycin A, 8'-Apo-β-caroten-8'-al, benzaldehyde, butanal, chloral, citral, citronellal, crotonaldehyde, dimethylamino benzaldehyde, folic acid, fosmidomycin, furfural, glutaraldehyde, glyceraldehyde, glycol aldehyde, glyoxal, glyoxylic acid, heptanal, 2-hydroxybenzaldehyde, 3-hydroxybutanal, hydroxymethylfurfural, 4-hydroxynonenal, isobutanal, isobutyraldehyde, methacrolein, 2-methylundecanal, mucochloric acid, N-methylformamide, 2-nitrobenzaldehyde, nonanal, octanal, oleocanthal, orlistat, pentanal, phenylethanal, phycocyanin, piperonal, propanal, propenal, protocatechualdehyde, retinal, salicylaldehyde, secologanin, streptomycin, strophanthidin, tylosin, vanillin, cinnamaldehyde and mixtures thereof.

5. The agent according to one of claims 1 to 4, **characterized in that** the first microcapsule contains phloroglucinol and the second microcapsule contains resorcinol as the aromatic alcohol a).

6. The agent according to claim 5, **characterized in that** it contains the first and the second microcapsule in a ratio of from 1:30 to 1:3, preferably 1:19 to 1:4 and particularly preferably 1:9 to 1:4.

7. The agent according to one of claims 1 to 6, **characterized in that** it contains the microcapsules mentioned in claim 1 in amounts of from 0.0001 to 50 wt.%, preferably 0.01 to 20 wt.%, and in particular 0.1 to 5 wt.%, based on the total agent.

8. The agent according to one of claims 1 to 7, **characterized in that** active substances, preferably odorants, are contained in the first and/or second microcapsules.

9. A method for releasing an active substance from a microcapsule located on a surface, the microcapsule, by contacting an agent for washing, cleaning, conditioning, maintaining and/or coloring hard or soft surfaces, comprising
i) at least one surfactant, and
ii) a mixture of at least one first and one second microcapsule which each comprise at least one active ingredient and of which the capsule walls each comprise a resin that can be obtained by reacting
a) at least one aromatic alcohol or an ether or derivatives thereof with
b) at least one aldehydic component that comprises at least two C atoms per molecule
c) in the presence of at least one (meth)acrylate polymer,
**characterized in that** the first and the second microcapsule differ from each other in at least one of the reacted components a) and/or b), and wherein the at least one aromatic alcohol is selected from resorcinol and/or phloroglucinol, is applied to the surface, and the release of the active substance subsequently takes place by means of mechanical force.

10. The method according to claim 9, **characterized in that** the active substance is released by means of friction.

11. The method according to one of claims 9 or 10, wherein the surface is a textile surface and the agent is a washing, cleaning or post-treatment agent.

## Revendications

1. Agent de lavage, de nettoyage, de conditionnement, d'entretien et/ou de coloration de surfaces dures ou souples, comprenant
i) au moins une substance tensioactive, et
ii) un mélange d'au moins une première et une deuxième microcapsule, dont les parois comprennent chacune une résine obtenue en faisant réagir
a) au moins un alcool aromatique ou son éther ou dérivé avec
b) au moins un composant aldéhyde composé d'au moins deux atomes de carbone par molécule, et
c) en présence d'au moins un polymère (méth)acrylate,
**caractérisé en ce que** la première et la seconde microcapsule diffèrent l'une de l'autre par au moins l'un des composants a) et/ou b) et dans lequel au moins un des alcools aromatiques est du résorcinol et/ou du phloroglucinol.

2. Agent selon la revendication 1, **caractérisé en ce que** la première et la seconde microcapsule diffèrent l'une de l'autre par les composants a) employés.

3. Agent selon la revendication 1 ou 2, **caractérisé en ce que** la première et la seconde microcapsule diffèrent l'une de l'autre par les composants b) employés.

4. Agent selon l'une des revendications 1 à 3, **caractérisé en ce que** le composant aldéhydique b) est choisi parmi le valéraldéhyde, le capronaldéhyde, le caprylaldéhyde, le décanal, le succindialdéhyde, le cyclohexane carbaldéhyde, le cyclopentane carbaldéhyde, le 2-méthyl-1-propanal, le 2-méthylpropionaldéhyde, l'acétaldéhyde, l'acroléine, l'aldostérone, l'antimycine A, le 8'-apo-β-carotène-8'-al, le benzaldéhyde, le butanal, le chloral, le citral, le citronellal, le crotonaldéhyde, le diméthylaminobenzaldéhyde, l'acide folique, le fosmidomycine, le furfural, le glutaraldéhyde, le glycéraldéhyde, l'aldéhyde glycolique, le glyoxal, l'acide glyoxylique, l'heptanal, le 2-hydroxybenzaldéhyde, le 3-hydroxybutanal, l'hydroxyméthylfurfural, le 4-hydroxynonenal, l'isobutanal, l'isobutyraldéhyde, la méthacroléine, le 2-méthylundecanal, l'acide mucochlorique, le N-methylformamide, le 2-nitrobenzaldehyde, le nonanal, l'octanal, l'oléocanthal, l'orlistate, le pentanal, le phényléthanal, la phycocyanine, le pipéronal, le propanal, le propénal, le protocatechualdéhyde, le rétinal, la salicylaldéhyde, la sécologanine, la streptomycine, la strophanthidine, la tylosine, la vanilline, l'aldéhyde cinnamique et leurs mélanges.

5. Agent selon l'une des revendications 1 à 4, **caractérisé en ce que** la première microcapsule contient de la phloroglucine et la seconde microcapsule contient du résorcinol en tant qu'alcool aromatique a).

6. Agent selon la revendication 5, **caractérisé en ce qu'**il contient la première et la deuxième microcapsule dans un rapport de 1:30 à 1:3, de préférence dans un rapport de 1:19 à 1:4 et en particulier de préférence dans un rapport de 1:9 à 1:4.

7. Agent selon l'une des revendications 1 à 6, **caractérisé en ce qu'**il contient les microcapsules mentionnées dans la revendication 1 en quantité allant de 0,0001 à 50 % en poids, de préférence de 0,01 à 20 % en poids, et en particulier de 0,1 à 5 % en poids par rapport à l'agent total.

8. Agent selon l'une des revendications 1 à 7, **caractérisé en ce que** la première et/ou la deuxième microcapsule contient des substances actives, de préférence des parfums.

9. Procédé pour libérer une substance active d'une microcapsule située sur une surface, dans lequel la microcapsule est obtenue en mettant en contact un agent pour laver, nettoyer, conditionner, entretenir et/ou colorer des surfaces dures ou souples comprenant
i) au moins un tensioactif et
ii) un mélange d'au moins une première et une seconde microcapsule contenant chacune au moins une substance active et dont les parois comprennent une résine obtenue par réaction
a) au moins un alcool aromatique ou son éther ou dérivé avec
b) au moins un composant aldéhyde composé d'au moins deux atomes de carbone par molécule, et
c) en présence d'au moins un polymère (méth)acrylate,
**caractérisé en ce que** la première et la seconde microcapsule diffèrent l'une de l'autre par au moins l'un des composants a) et/ou b) et dans lequel au moins un des alcools aromatiques est du résorcinol et/ou du phloroglucinol, sont appliquées à la surface et y libèrent la substance active qu'elles contiennent par force mécanique.

10. Procédé selon la revendication 9, **caractérisé en ce que** la libération de la substance active s'effectue par friction.

11. Procédé selon l'une des revendications 9 ou 10, dans lequel la surface est une surface textile et l'agent est un agent de lavage, de nettoyage ou de post-traitement.
